# EUROPEAN PATENT SPECIFICATION

(11) **EP 1 452 586 B1**
(45) Date of publication and mention of the grant of the patent: **30.05.2012**
(21) Application number: 02775435.7
(22) Date of filing: 30.10.2002
(51) Int. Cl.: C12N 5/079, A61K 35/28, A61P 25/00

(54) **METHOD OF INDUCING DIFFERENTIATION OF MESODERMAL STEM CELLS INTO NERVOUS SYSTEM CELLS**
VERFAHREN ZUR INDUKTION DER DIFFERENZIERUNG MESODERMALER STAMMZELLEN ZU ZELLEN DES NERVENSYSTEMS
PROCEDE PERMETTANT D'INDUIRE UNE DIFFERENCIATION DE CELLULES SOUCHES EMBROYONNAIRES MESODERMIQUES DANS DES CELLULES DU SYSTEME NERVEUX

(30) Priority: 30.10.2001 WO PCT/JP01/09510; 03.04.2002 WO PCT/JP02/03344
(43) Date of publication of application: 01.09.2004
(73) Proprietor: NC MEDICAL RESEARCH INC., 4-3-1 Toranomon Minato-ku, Tokyo 105-6017 (JP); ASKA Pharmaceutical Co., Ltd., Tokyo 108-8532 (JP); Mitsui Sumitomo Insurance Care Network Company, Limited, Chiyoda-ku Tokyo 101-0052 (JP); Hitachi, Ltd., Tokyo 100-8280 (JP)
(72) Inventor: HONMOU, Osamu, Sapporo-shi, Hokkaido 064-0801 (JP); HAMADA, Hirofumi, Sapporo-shi, Hokkaido 064-0959 (JP)
(74) Representative: Vossius & Partner
(86) International application number: PCT/JP2002/011294
(87) International publication number: WO 2003/038075

(56) References cited:
- WO-A-00/69448
- WO-A-01/59072
- WO-A-01/68815
- WO-A-01/78753
- WO-A-99/56759
- DE-C1- 19 756 864
- WOODBURY D ET AL: "ADULT RAT AND HUMAN BONE MARROW STROMAL CELLS DIFFERENTIATE INTO NEURONS" JOURNAL OF NEUROSCIENCE RESEARCH, WILEY-LISS, US, vol. 61, 2000, pages 364-370, XP002940452 ISSN: 0360-4012
- DENG WEIWEN ET AL: "In vitro differentiation of human marrow stromal cells into early progenitors of neural cells by conditions that increase intracellular cyclic AMP" BIOCHEMICAL AND BIOPHYSICAL RESEARCH COMMUNICATIONS, ACADEMIC PRESS, SAN DIEGO, CA, US, vol. 282, no. 1, 23 March 2001 (2001-03-23), pages 148-152, XP002199140 ISSN: 0006-291X
- SANCHEZ-RAMOS J ET AL: "Adult bone marrow stromal cells differentiate into neural cells in vitro" EXPERIMENTAL NEUROLOGY, vol. 164, no. 2, August 2000 (2000-08), pages 247-256, XP002297662 ISSN: 0014-4886
- FRAICHARD A ET AL: "In vitro differentiation of embryonic stem cells into glial cells and functional neurons" JOURNAL OF CELL SCIENCE, vol. 108, no. 10, 1995, pages 3181-3188, XP002317724 ISSN: 0021-9533
- BAIN GERARD ET AL: "Embryonic stem cells express neuronal properties in vitro" DEVELOPMENTAL BIOLOGY, vol. 168, no. 2, 1995, pages 342-357, XP002318491 ISSN: 0012-1606
- BRUSTLE O ET AL: "In vitro-generated neural precursors participate in mammalian brain development" PROCEEDINGS OF THE NATIONAL ACADEMY OF SCIENCES OF USA, NATIONAL ACADEMY OF SCIENCE. WASHINGTON, US, vol. 94, December 1997 (1997-12), pages 14809-14814, XP002105104 ISSN: 0027-8424
- REUBINOFF BENJAMIN E ET AL: "Embryonic stem cell lines from human blastocysts: Somatic differentiation in vitro" NATURE BIOTECHNOLOGY, NATURE PUB. CO, NEW YORK, NY, US, vol. 18, no. 4, April 2000 (2000-04), pages 399-404, XP002195338 ISSN: 1087-0156
- REYES MORAYMA ET AL: "In vitro and in vivo characterization of neural cells derived from mesenchymal stem cells" BLOOD, vol. 96, no. 11 Part 1, 16 November 2000 (2000-11-16), page 494a, XP009044219 & 42ND ANNUAL MEETING OF THE AMERICAN SOCIETY OF HEMATOLOGY; SAN FRANCISCO, CALIFORNIA, USA; DECEMBER 01-05, 2000 ISSN: 0006-4971
- REYES M ET AL: "EX VIVO DIFFERENTIATION OF MESENCHYMAL STEM CELLS INTO OLIGODENDROCYTES, ASTROCYTES, AND/OR DOPAMINERGIC, GAMMA-AMINO-BUTYRIC-ACID-ERGIC OR SEROTONERGIC NEURONS" ABSTRACTS OF THE SOCIETY FOR NEUROSCIENCE, SOCIETY FOR NEUROSCIENCE, WASHINGTON, DC, US, vol. 26, no. 1/2, 2000, page 1101, XP009036838 ISSN: 0190-5295
- OLIVER L J ET AL: "Long-term culture of human bone marrow stromal cells in the presence of basic fibroblast growth factor." GROWTH FACTORS (CHUR, SWITZERLAND) 1990, vol. 3, no. 3, 1990, pages 231-236, XP002318492 ISSN: 0897-7194
- REYES M ET AL: "TURNING MARROW INTO BRAIN: GENERATION OF GLIAL AND NEURONAL CELLS FROM ADULT BONE MARROW MESENCHYMAL STEM CELLS" BLOOD, W.B.SAUNDERS COMPANY, ORLANDO, FL, US, vol. 94, no. 10,SUPP01PART01, 15 November 1999 (1999-11-15), page 377A, XP009007196 ISSN: 0006-4971
- SONG S ET AL: "Study of optimal conditions for differentiation of neural cells from bone marrow precursors" SOCIETY FOR NEUROSCIENCE ABSTRACTS, vol. 26, no. 1-2, 2000, pages Abstract No.-312.20, XP001205338 & 30TH ANNUAL MEETING OF THE SOCIETY OF NEUROSCIENCE; NEW ORLEANS, LA, USA; NOVEMBER 04-09, 2000 ISSN: 0190-5295
- OKABE S ET AL: "DEVELOPMENT OF NEURONAL PRECURSOR CELLS AND FUNCTIONAL POSTMITOTIC NEURONS FROM EMBRYONIC STEM CELLS IN VITRO" MECHANISMS OF DEVELOPMENT, ELSEVIER SCIENCE IRELAND LTD, IE, vol. 59, 1996, pages 89-102, XP001052878 ISSN: 0925-4773
- REUBINOFF B E ET AL: "Neural progenitors from human embryonic stem cells" NATURE BIOTECHNOLOGY, NATURE PUBLISHING, US, vol. 19, no. 12, December 2001 (2001-12), pages 1134-1140, XP002971362 ISSN: 1087-0156
- SANG-HUN LEE ET AL: "EFFICIENT GENERATION OF MIDBRAIN AND HINDBRAIN NEURONS FROM MOUSE EMBRYONIC STEM CELLS" NATURE BIOTECHNOLOGY, NATURE PUB. CO, NEW YORK, NY, US, vol. 18, June 2000 (2000-06), pages 675-679, XP000941668 ISSN: 1087-0156
- SCHULDINER MAYA ET AL: "Effects of eight growth factors on the differentiation of cells derived from human embryonic stem cells" PROCEEDINGS OF THE NATIONAL ACADEMY OF SCIENCES OF USA, NATIONAL ACADEMY OF SCIENCE. WASHINGTON, US, vol. 97, no. 21, 10 October 2000 (2000-10-10), pages 11307-11312, XP002184277 ISSN: 0027-8424
- THOMAS ET AL: "Fibroblast growth factors" FASEB JOURNAL, FED. OF AMERICAN SOC. FOR EXPERIMENTAL BIOLOGY, BETHESDA, MD, US, vol. 1, no. 6, December 1987 (1987-12), pages 434-440, XP002138001 ISSN: 0892-6638
- GRITTI A ET AL: "MULTIPOTENTIAL STEM CELLS FROM THE ADULT MOUSE BRAIN PROLIFERATE AND SELF-RENEW IN RESPONSE TO BASIC FIBROBLAST GROWTH FACTOR" JOURNAL OF NEUROSCIENCE, NEW YORK, NY, US, vol. 16, no. 3, 1 February 1996 (1996-02-01), pages 1091-1100, XP000881158 ISSN: 0270-6474
- KILPATRICK T J ET AL: "CLONING AND GROWTH OF MULTIPOTENTIAL NEURAL PRECURSORS: REQUIREMENTS FOR PROLIFERATION AND DIFFERENTIATION" NEURON, CAMBRIDGE, MA, US, vol. 10, 1992, pages 255-265, XP000916281
- FLAX JONATHAN D ET AL: "Engraftable human neural stem cells respond to developmental cues, replace neurons, and express foreign genes" NATURE BIOTECHNOLOGY, NATURE PUBLISHING, US, vol. 16, no. 11, November 1998 (1998-11), pages 1033-1039, XP002197601 ISSN: 1087-0156
- VESCOVI A L ET AL: "ISOLATION AND CLONING OF MULTIPOTENTIAL STEM CELLS FROM THE EMBRYONIC HUMAN CNS AND ESTABLISHMENT OF TRANSPLANTABLE HUMAN NEURAL STEM CELL BY EPIGENETIC STIMULATION" EXPERIMENTAL NEUROLOGY, ACADEMIC PRESS, NEW YORK, NY, US, vol. 156, 1999, pages 71-83, XP001126942 ISSN: 0014-4886
- KIMURA A ET AL: "EFFECTS OF PLATELET DERIVED GROWTH FACTOR EPIDERMAL GROWTH FACTOR AND TRANSFORMING GROWTH FACTOR-BETA ON THE GROWTH OF HUMAN MARROW FIBROBLASTS" BRITISH JOURNAL OF HAEMATOLOGY, vol. 69, no. 1, 1988, pages 9-12, XP009044262 ISSN: 0007-1048
- KORNBLUM H I ET AL: "EPIDERMAL GROWTH FACTOR AND BASIC FIBROBLAST GROWTH FACTOR EFFECTS ON AN OVERLAPPING POPULATION OF NEOCORTICAL NEURONS IN-VITRO" BRAIN RESEARCH, vol. 535, no. 2, 1990, pages 255-263, XP002318497 ISSN: 0006-8993
- RHYU M S: "TELOMERES, TELOMERASE, AND IMMORTALITY" JOURNAL OF THE NATIONAL CANCER INSTITUTE, US DEPT. OF HEALTH, EDICATIONAND WELFARE, PUBLIC HEALTH, US, vol. 87, no. 12, 21 June 1995 (1995-06-21), pages 884-894, XP000199529 ISSN: 0027-8874
- KIM N W ET AL: "SPECIFIC ASSOCIATION OF HUMAN TELOMERASE ACTIVITY WITH IMMORTAL CELLS AND CANCER" SCIENCE, AMERICAN ASSOCIATION FOR THE ADVANCEMENT OF SCIENCE,, US, vol. 266, 23 December 1994 (1994-12-23), pages 2011-2015, XP002028668 ISSN: 0036-8075
- SASAKI M. ET AL.: 'Transplantation of an acutely isolated bone marrow fraction repairs demyelinated adult rat spinal cord axons' GLIA vol. 35, no. 1, 2001, pages 26 - 34, XP002945994
- TREMAIN N. ET AL.: 'MicroSAGE analysis of 2,353 expresed genes in a single cell-derived colony of undifferentiated human mesenchymal stem cells reveals mRNAs of multiple cell lineages' STEM CELLS vol. 19, 2001, pages 408 - 418, XP002907879
- BRUSTLE O. ET AL.: 'Embryonic stem cell-derived glial precursors: a source of myelinating transplants' SCIENCE vol. 285, 1999, pages 754 - 756, XP002906546
- PITTENGER M.F. ET AL.: 'Multilineage potential of adult human mesenchymal stem cells' SCIENCE vol. 284, 1999, pages 143 - 147, XP002936517

## Description

### Technical Field

The present invention relates to a method for inducing differentiation of immortalized mesodermal stem cells into neural cells and uses thereof.

### Background Art

Transplantation of oligodendrocytes (i.e., oligodendroglia) (Archer D. R. et aL, 1994, Exp. Neurol. 125:268-77; Blakemore W. F., and Crang A. J., 1988, Dev. Neurosci. 10:1-11; Gumpel M. et aL 1987, Ann. New York Acad. Sci. 495:71-85) or myelin-forming cells, such as Schwann cells (Blakemore W. F., 1977, Nature 266:68-9; Blakemore W. F., and Crang A. J., 1988, Dev. Neurosci. 10:1-11; Honmou O. et al., 1996, J. Neurosci. 16:3199-208) or olfactory ensheating cells (Franklin R. J. et al. 1996, Glia 17:217-24; Imaizumi T. et al., 1998, J. Neurosci. 18(16):6176-6185; Kato T. et aL, 2000, Glia 30:209-218), can elicit remyelination in animal models and recovery of electrophysiological function (Utzschneider D. A. et al., 1994, Proc. Natl. Acad. Sci. USA. 91:53-7; Honmou O. et aL, 1996, J. Neurosci. 16:3199-208). It is possible to prepare such cells from patients or other persons for cell therapy. However, this method is considerably problematic because tissue material must be collected from either the brain or nerves.

Neural progenitor cells or stem cells derived from brain have self-reproducing ability and can differentiate into neurons and glia cells of various lineages (Gage F.H. et al. 1995, Proc. Natl. Acad. Sci. USA. 92:11879-83; Lois C. and Alvarez-Buylla A., 1993, Proc. Natl. Acad. Sci. USA. 90:2074-7; Morshead C. M. et al., 1994, Neuron 13:1071-82; Reynolds B. A., and Weiss S., 1992, Science 255:1707-10). Upon transplantation into newborn mouse brain, human neural stem cells collected from fetal tissues differentiate into neurons and astrocytes (Chalmers-Redman R.M. et al., 1997. Neurosci. 76:1121-8; Moyer M. P. et al., 1997, Transplant. Proc. 29:2040-1; Svendsen C.N. et al., 1997, Exp. Neurol. 148:135-46), and myelinate the axons (Flax J. D. et al., 1998, Nat. Biotechnol. 16:1033-9). Remyelination and recovery of impulse conduction upon transplantation of neural progenitor (stern) cells derived from adult human brain into demyelinated rodent spinal cord have been reported (Akiyama Y et al., 2001, Exp. Neurol. 167:27-39).

These studies have evoked great interest due to the indicated possibility of the application of the above-mentioned cells in regenerative strategies for neurological diseases (Akiyama Y et al., 2001, Exp. Neurol. 167:27-39: Chalmers-Redman R. M. et al., 1997, Neurosci. 76:1121-8; Moyer M. P. et al., 1997, Transplant. Proc. 29:2040-1; Svendsen C. N. et al., 1997, Exp. Neurol. 148:135-46; Yandava B. D. et al., 1999, Proc. Natl. Acad. Sci. USA 96:7029-34).

The present inventors previously isolated and cultured neural stem cells from adult human brain, and established some cell lines. By studying their functions, the inventors discovered that neural stem cells have pluripotency and self-reproducing ability (Akiyama Y et al., 2001. Exp. Neurol. 167:27-39). Specifically, single-cell expansion of neural progenitor (stem) cells obtained from adult human brain was conducted to establish cell lines; the established cells were then subjected to *in vivo* clonal analysis. The results demonstrated that the cell lines had pluripotency (namely, the ability to differentiate into neuron, astroglia (or astrocyte), and oligodendroglia (i.e., oligodendrocyte)) and self-reproducing ability (namely, proliferation potency). Thus, these cells were confirmed to possess the characteristics of neural stem cells.

Transplantation of these cells indeed resulted in very favorable graft survival, migration, and differentiation in cerebral ischemic model rats or traumatic model rats. Furthermore, transplantation of the cells resulted in functional myelin sheath formation in spinal cord demyelination model rats. Thus, such transplantation allows remyelination of the demyelinated axon and restoration of the neural function in the rat spinal cord demyelination model. Effectiveness of such transplantation therapy using these cells was confirmed by histological, electrophysiological, and behavior studies.

Judging from the above-described findings, transplantation of cultured neural stem cells, which have been isolated from a small amount of neural tissue collected from the cerebrum of a patient, into a patient's spinal cord lesion appears to be widely applicable in autotransplantation therapy.

However, it is difficult to collect tissues containing neural stem cells from the cerebrum, despite the fact that such a collection does not result in neurological deficit. Therefore, it is very important to establish a safer and more convenient method for autotransplantation therapy, from the standpoint of establishment of methods for treating various complex diseases of today. Thus, the present inventors have developed a simplified technique for preparing donor cells involving the collection of mononuclear cell fractions or the like from bone marrow cells, umbilical blood cells, or fetal hepatocytes (Patent Application No. 2001-160579); this technique is much easier as compared to prior art methods that require collection of neural stem cells. Specifically, the present inventors discovered that a mononuclear cell fraction prepared from bone marrow cells has the ability to differentiate into neural cells. Furthermore, the present inventors found that a cell fraction containing mesodermal stem cells isolated from the mononuclear cell fraction, a cell fraction containing interstitial cells, and a cell fraction containing AC133-positive cells also have the ability to differentiate into neural cells.

### Disclosure of the Invention

The present invention was made in view of these circumstances. The objective of the present invention is to provide a method for inducing differentiation of mesodermal stem cells into neural cells, a method which comprises preparing a sufficient number of cells by allowing proliferation of mesodermal stem cells and efficiently inducing differentiation of the cells into neural stem cells and neural cells; neural cells obtained by the methods; a composition for treating neurological diseases which composition comprises the neural cells; and a method for treating neurological diseases using the composition.

The present inventors newly discovered that mesodermal stem cells in a mononuclear cell fraction isolated from bone marrow fluid or umbilical blood can differentiate into neural cells *in vitro.*

Specifically, the inventors discovered that when the mesodermal stem cells prepared from the mononuclear cell fraction isolated from bone marrow fluid or umbilical blood are cultured in suspension under 5% CO₂ at 37°C in culture medium 1 [50% DMEM (Dulbecco's modified essential medium)/50% F-12/1% FSC; 10 ng/ml bFGF (basic fibroblast growth factor) and 10 ng/ml EGF (epidermal growth factor) added every day] or culture medium 2 [NPBM (Neural Progenitor Basal Medium)/2% neural survival factors (Clonetics)/0.2% hEGF (human epidermal growth factor)/0.2% Gentamicin-amphotericin B/0.2% hFGF (human fibroblast growth factor); 10 ng/ml bFGF and 10 ng/ml EGF added every day], the mesodermal stem cells differentiated into neural stem cells.

The inventors also found that, when the mesodermal stem cells in the mononuclear cell fraction isolated from bone marrow fluid or umbilical blood are cultured in culture medium 1 (50% DMEM/50% F-12/1% FSC) or culture medium 2 [NPBM (Neural progenitor cell basal medium; Clonetics)/2% Neural survival factors (Clonetics)/0.2% hEGF (human epidermal growth factor)/0.2% Gentamicin-amphotericin B/0.2% hFGF (human fibroblast growth factor)], the mesodermal stem cell differentiated into neuron or glial cells.

Furthermore, the inventors discovered that the differentiation of the mesodermal stem cells into neural cells could be further promoted by adding ischemic brain extract to the above-mentioned culture medium.

In addition, the neural regeneration potency of the neural cells obtained by the above-mentioned method for inducing differentiation was evaluated using a brain infarction model, a dementia model, a spinal cord injury model and a demyelination model. As a result, the cells were revealed to have regeneration potency comparable to that of neural stem cells extracted and cultured from the brain.

Based on the above findings, neural cells obtained by the above-described method for inducing differentiation can be used to treat brain infarction, dementia, spinal cord injury, demyelination diseases, and the like. In addition, the present invention is considered to be applicable to neural transplantation and/or regeneration therapy for more general and diffuse cerebral and/or nerve damages. Specifically, the present invention can be used in autotransplantation therapy for ischemic cerebral and/or nerve injury of the central nervous system and peripheral nervous system, traumatic cerebral and/or nerve injury, brain neurological degenerative diseases and metabolic neurological diseases.

Furthermore, the above-mentioned method for inducing differentiation provides may help elucidate the mechanism underlying the differentiation of mesodermal stem cells into neural cells. Once genes that direct such differentiation are identified and analyzed, a sufficient number of mesodermal stem cells can be transformed efficiently into neural cells using the genes. Thus, the invention shows great promise to establish a method of "gene therapy" for promoting regeneration of neural tissues.

Moreover, the present inventors succeeded in developing a method for inducing differentiation in the nervous system which ensures stable proliferation of a large number of cells. Generally, mesodermal stem cells are useful in the medical field of neural regeneration; however, the proliferation of such cells is limited to some extent under culture conditions. However, according to the study of the present inventors, introduction of a viral vector containing as an insert an immortalization gene, such as telomerase, into stromal cells *in vitro* was revealed to result in the continuation of cell proliferation of the host cell, even after cycles of cell division, with greatly extended life span of the cells retaining the same morphology as normal cells. The present inventors focused on the fact that a large number of cells could be stably proliferated by immortalizing mesodermal stem cells through the introduction of an immortalization gene into the cells. Furthermore, the inventors continued their study to discover that differentiation of the mesodermal stem cells, which had been immortalized by introducing an immortalization gene into the cells, into neural stem cells and neural cells can be efficiently induced under an appropriate culture condition.

Specifically, the inventors succeeded in inducing differentiation of mesodermal stem cells, which had been immortalized through the introduction of the immortalization gene hTERT, into fat cells, chondroblasts, osteoblasts, and the like. Furthermore, the inventors induced differentiation of mesodermal stem cells immortalized by the introduction of the hTERT gene into neural cells containing neural stem cells at a high percentage. The present inventors further revealed that demyelinated areas in the spinal cord can be repaired by transplanting these cells (mesodermal stem cells themselves; neural stem cells differentiated from the mesodermal stem cells; neural cells differentiated from neural stem cells which had been differentiated from the mesodermal stem cells; and neural cells differentiated from the mesodermal stem cells) into the demyelinated areas.

In addition, neural stem cells and neural cells whose differentiation is induced according to the above-described method of the present invention, from mesodermal stem cells having an immortalization gene introduced therein, are expected to be very useful in achieving neural regeneration.

When a cell is immortalized through introduction of an *oncogene* or the like, the character of the cell is also transformed. On the contrary, when a cell is immortalized through introduction of an *immortalization* gene as in the present invention, the cell retains its original character. In addition, in cases where an immortalization gene has been introduced, the gene can be removed after sufficiently proliferating the cell.

As described above, the present inventors developed a novel method for efficiently inducing differentiation of mesodermal stem cells into neural cells, and thus completed the invention.

Specifically, the present invention relates to a method for inducing differentiation of mesodermal stem cells into neural cells; a method for inducing efficient differentiation of mesodermal stem cells into neural cells, including neural stem cells, which comprises immortalization of the mesodermal stem cells; a neural cell obtained by the methods; a composition for treating neurological diseases which comprises the neural cell; and a method for treating neurological diseases using the composition. More specifically, the present invention relates to:
[1] a method for inducing differentiation of a mesodermal stem cell derived from a mononuclear cell fraction isolated from bone marrow fluid or umbilical blood collected from a vertebrate into a neural cell by incubating the cell in a basal culture medium at 33°C to 38°C;
[2] a method for inducing differentiation of a mesodermal stem cell into a neural cell, which comprises the steps of:
   (a) providing an immortalized mesodermal stem cell by highly expressing or activating an immortalization gene; and
   (b) inducing the differentiation of the immortalized mesodermal stem cell of step (a) into a neural cell by culturing the mesodermal stem cell in a basal culture medium;
[3] the method according to [2], wherein the immortalization gene is highly expressed or activated through the introduction of the immortalization gene into the mesodermal stem cell;
[4] the method according to [3], wherein the mesodermal stem cell is immortalized by introducing the immortalization gene into the mesodermal stem cell using a retroviral vector;
[5] the method according to [4], wherein the retroviral vector is pBabe;
[6] the method according to any one of [2] to [5], wherein the immortalization gene of the neural cell can be or has been removed by a gene-removing treatment;
[7] the method according to [6], wherein the gene-removing treatment of the immortalization gene is performed by sandwiching the immortalization gene within a pair of loxP sequences or loxP-like sequences and then treating with a recombinase;
[8] the method according to any one of [2] to [7], wherein the immortalization gene is any one selected from the group of a telomerase gene, genes derived from telomerase, and genes which regulate the expression or activity of telomerase;
[9] the method according to any one of [2] to [8], wherein the mesodermal stem cell is derived from bone marrow fluid, umbilical blood, peripheral blood, skin, hair root cells of skin, muscle tissue;
[10] the method according to any one of [2] to [8], wherein the mesodermal stem cell is contained in a mononuclear cell friction isolated from bone marrow fluid or umbilical blood collected from a vertebrate;
[11] the method according to [1] or [10], wherein the mononuclear cell fraction can be prepared by subjecting the bone marrow fluid or umbilical blood collected from a vertebrate to density-gradient centrifugation in a solution at 2000 rpm for a sufficient time ensuring separation depending on the specific gravity, and then recovering a cell fraction having a specific gravity ranging from 1.07 g/ml to 1.1 g/ml;
[12] the method according to any one of [1] to [11], wherein the mesodermal stem cell has the characteristics of SH2(+), SH3(+), SH4(+), CD29(+), CD44(+), CD14(-), CD34(-), and CD45(-);
[13] the method according to any one of [2] to [12], wherein the culture is carried out at 33°C to 38°C;
[14] the method according to any one of [1] to [13], which further comprises adding bFGF, EGF, or ischemic brain extract to a basal culture medium;
[15] the method according to any one of [1] to [14], wherein the neural cell is selected from the group consisting of neural stem cell, neural progenitor cell, neuron, and glial cell;

First, the present invention provides a method for inducing differentiation of a mesodermal stem cell derived from a mononuclear cell fraction isolated from bone marrow fluid or umbilical blood collected from a vertebrate into neural cells by incubating the mesodermal stem cell, in a basal culture medium at 33°C to 38°C.

The present invention also provides a novel method for efficiently inducing differentiation of a mesodermal stem cell into neural stem cells and neural cells, which method comprises the step of preparing a large number of mesodermal stem cells by introducing an immortalization gene into the mesodermal stem cell. In a preferred embodiment of the present invention, the mesodermal stem cell is immortalized by highly expressing or activating an immortalization gene in the mesodermal stem cell, and then inducing differentiation of the mesodermal stem cell into neural cells by culturing the cell.

According to the method of the present invention described above, first, an immortalized mesodermal stem cell is provided by highly expressing or activating the immortalization gene (step (a)).

As used herein, the term "mesodermal stem cell" refers to a cell constituting tissues embryologically categorized into the class of mesoderm, including blood cells. A "mesodermal stem cell" is also a cell which can make copies of itself (division and proliferation) having the same potency as that of the original cell, and which has the ability to differentiate into all cell types constituting mesodermal tissues. The mesodermal stem cell expresses, for example, the cell markers SH2(+), SH3(+), SH4(+), CD29(+), CD44(+), CD14(-), CD34(-), and CD45(-), but such cells are not limited to these markers. Furthermore, so-called mesenchyme-related stem cell is also included in the mesodermal stem cells of the present invention.

The term "mesenchyme-related cell" refers to mesenchymal stem cells, mesenchymal cells, precursor cells of mesenchymal cells and cells derived from mesenchymal cells.

The term "mesenchymal stem cell" refers to stem cells that can be obtained from bone marrow, peripheral blood, skin, hair root, muscle tissue, uterine endometrium, blood, umbilical blood and primary cultures of various tissues.

The phrase "precursor cells of mesenchymal cell" refers to cells that differentiate from a mesenchymal stem cell and are on the way to differentiating into mesenchymal cells.

A mesenchymal cell is generated by the differentiation of a mesenchymal stem cell. Unlike the stem cells, the mesenchymal cell has no pluripotency. However, the mesenchymal cell has differentiation potency in limited directions as well as proliferation potency. Normally, the mesenchymal cell is arrested in the G₀ phase, but can proceed to the G₁ phase (start of division) upon stimulation. The mesenchymal cell includes, for example, stromal cell and cells having the nature of stromal cell. The mesenchymal cell is present in every organ including, for example, subcutaneous tissue, lung and liver, and exists in mesenchymal tissues, such as bone, cartilage, fat, tendon, skeletal muscle and stroma of bone marrow.

The cells derived from mesenchymal cells encompass: (1) cells of the cardiovascular system, such as endothelial cells and cardiac muscle cells, precursor cells of the cells of the cardiovascular system, and cells having the characteristics of these cells; (2) any cells of bone, cartilage, tendon and skeletal muscle, precursor cells of any cells of bone, cartilage, tendon, skeletal muscle and fat tissue, and cells having the characteristics of such cells; (3) cells of the nervous system (neural cells), precursor cells thereof and cells having the characteristics of such cells; (4) endocrine cells, precursor cells thereof and cells having the characteristics of such cells; (5) hematopoietic cells, precursor cells thereof and cells having the characteristics of such cells; and (6) hepatocytes, precursor cells thereof and cells having the characteristics of such cells.

A mesodermal stem cell of the present invention can be prepared from cells derived, for example, from bone marrow fluid, umbilical blood, peripheral blood, skin, hair root cells of skin, muscle tissue of vertebrates. Such cells can be prepared from the skin, for example, according to the method of Young et al. (Young et al., 2001, Anat. Rec. 264(1):51-62; Campagnli et al., 2001, Blood 98(8):2396-2402); from the muscle, for example, according to the method of Asakura et al. (Asakura A et al., 2001, Differentiation 68(4-5):245-253); from a fat tissue, for example, according to the method of Zuk et al. (Zuk PA et al.,2001. Tissue Eng. 7(2):211-228); and from the synovial membrane, for example, according to the method of De Bari et al. (De Bari C et al., 2001, Arthritis Rheum. 44(8):1928-1942).

In the context of the present invention, preferred vertebrates include mammals (for example, mouse, rat, rabbit, pig, dog, monkey, human, etc.), but the invention is not restricted thereto.

The bone marrow fluid to be used in the present invention can be collected, for example, by anesthetizing (local or systemic anesthesia) a vertebrate animal (including human), puncturing a bone and aspirating cells with a syringe. A suitable source bone includes, but is not limited to, for example, thigh bone, sternum and iliac bone forming the pelvis. Further, a procedure that involves directly puncturing the umbilical cord and aspirating blood with a syringe to collect and store the umbilical blood at birth has become an established technique.

According to the present invention, the mesodermal stem cells can be prepared by subjecting bone marrow fluid or umbilical blood collected from a vertebrate to density-gradient centrifugation in a solution at 900 g for a sufficient time ensuring separation depending on the specific gravity, and then recovering a cell fraction of a specific gravity within the range of 1.07 g/ml to 1.1 g/ml. As used herein, the phrase "sufficient time ensuring separation depending on the specific gravity" refers to a time sufficient for the cells to reach a position depending on their specific gravity in a solution of density-gradient centrifugation, which is typically about 10 to about 30 minutes. The specific gravity of cells to be collected can be altered depending on the type of animal from which the cells are derived (for example, human, rat, mouse, etc.). Solutions to be used in density-gradient centrifugation include, but are not limited to, Ficoll solution and Percoll solution.

A specific example is as follows. Bone marrow fluid (25 ml) or umbilical blood collected from a vertebrate animal is combined with an equal volume of PBS. The mixture is centrifuged at 900 g for 10 minutes. Precipitated cells are recovered by adding PBS to the cells (cell density = about 4x 10⁷ cells/ml) to remove other blood components. Then, a 5-ml aliquot of the cell suspension is combined with Percoll solution (1.073 g/ml), and centrifuged at 900 g for 30 minutes to extract a mononuclear cell fraction. To wash the cells, the extracted mononuclear cell fraction is combined, for example, with culture medium 1 [DMEM (Dulbecco's Modified Eagles Medium-Low Glucose)/10% FBS (fetal bovine serum)/1% anti-biotic-antimycotic solution], culture medium 2 [MSCBM (Mesenchymal Stem Cell Basal Medium)/10% MCGS (Mesenchymal Cell Growth Supplement)/4 mM L-glutamine/1% penicillin-streptomycin], or culture medium 3 [DMEM (Sigma)/10% FBS (gibco)/1% Penicillin-Streptomycin/2 mM L-Glutamine (Gibco)], and then centrifuged at 900 g for 15 minutes or at 2,000 rpm for 15 minutes. Then, the supernatant after centrifugation is discarded and the cells precipitated are collected to culture the cells at 37°C under 5% CO₂ atmosphere.

The mesodermal stem cells can also be obtained, for example, by selecting cells having the above-mentioned cell-surface markers, namely, SH2(+), SH3(+), SH4(+), CD29(+), CD44(+), CD14(-), CD34(-), CD45(-), and such from the above-mentioned mononuclear cell fraction using antibodies. There is no limitation on the method of selection and methods using magnetic beads or typical cell sorters (FACS, etc.) are included herein.

Alternatively, according to the present invention, the mesodermal stem cells can be prepared from the mononuclear cell fraction (including those in a culture solution). The mononuclear cell fraction can be prepared by performing density-gradient centrifugation of bone marrow fluid or umbilical blood collected from a vertebrate animal at 900 g for a sufficient time ensuring separation depending on the specific gravity in a solution, and then recovering a cell fraction within the range of specific gravity, 1.07 g/ml to 1.1 g/ml. As used herein, the phrase a "sufficient time ensuring separation depending on the specific gravity" refers to a time sufficient for the cells to reach a position depending on their specific gravity in a solution of density-gradient centrifugation, which is typically about 10 to about 30 minutes. The specific gravity of the cell fraction to be recovered preferably falls within the range of 1.07 g/ml to 1.08 g/ml (for example, 1.077 g/ml). Solutions to be used in density-gradient centrifugation include but are not limited to Ficoll solution and Percoll solution.

A specific example is as follows. First, bone marrow fluid (5-10 µl) collected from a vertebrate is combined with a solution (2 ml of L-15 and 3 ml of Ficoll). Then, mononuclear cell fraction (about 1 ml) is extracted by centrifuging the mixture at 900 g for 15 minutes. The mononuclear cell fraction is combined with a culture solution (2 ml of NPBM) and centrifuged again at 900 g for 15 minutes to wash the cells. Then, the supernatant is discarded and the precipitated cells are collected.

A culture solution of the mononuclear cell fraction of the present invention may also be used to prepare the mesodermal stem cells as described below. Such a culture solution containing the mononuclear cell fraction can be prepared, for example, by culturing the cells of the mononuclear cell fraction in the above-mentioned culture medium 1, culture medium 2, or culture medium 3 at 37°C under 5% CO₂, though the present invention is not limited to the specific culture conditions.

There is no limitation on the type of neural cells of the present invention and the present invention includes neural stem cells, neural progenitor cells, neurons, and glial cells.

As used herein, the term "cell immortalization" refers to a state wherein a cell continuously proliferates even after a certain number of cell divisions, whereas normally, cells stop proliferation after a certain number of divisions. Thus, as used herein, the term "immortalization gene" refers to a gene that directs the cell to continue cell division even after such number of cell divisions. Such genes include, but are not limited to, telomerase genes, genes derived from a telomerase, and genes that regulate the expression or activity of a telomerase (for example, the myc gene has been reported to enhance telomerase activity). In the present invention, the human telomerase gene (hTERT) is a particularly preferred embodiment.

According to the present invention, methods for highly expressing or activating an immortalization gene include, but are not limited to, a method wherein the immortalization gene is introduced into a mesodermal stem cell. The introduction of an immortalization gene into a mesodermal stem cell can be conducted by any of various methods known in the art. Exemplary methods include a method wherein an immortalization gene is inserted into a plasmid vector and then the vector is introduced for transformation into a mesodermal stem cell in the presence of calcium phosphate; a method wherein an immortalization gene is introduced into a mesodermal stem cell by contacting the gene, in conjunction with a vesicle such as liposome, with the cells; a method wherein an immortalization gene is introduced into a mesodermal stem cell by electroporation; and a method wherein an immortalization gene is introduced into various viral vectors and the vector is then infected to a mesodermal stem cell for gene transfer. The method using a viral vector includes methods using retrovirus, adenovirus and adeno-associated virus; and the method using the retroviral vector includes the method using MoMLV virus, and the like. In the present invention, the pBabe vector can be preferably used.

The step of immortalizing mesodermal stem cells by the above-described method is not essential to the present invention and mesodermal stem cells previously immortalized by the above-described method or the like may be used in step (b) described below.

According to the method of the present invention, following step (a) described above, mesodermal stem cells are differentiated into neural cells by culturing the immortalized mesodermal stem cells in a basal culture medium (step (b)).

In a preferred embodiment of this step, the mesodermal stem cells are differentiated into neural stem cells by culturing the immortalized mesodermal stem cells in a basal culture medium.

In a preferred embodiment of the present invention, the above-mentioned mesodermal stem cells are incubated in the basal culture medium at 33°C to 38°C.

There is no limitation on the type of the basal culture medium to be used in the present invention, so long as it is a typical culture medium that can be used for cell culture. Preferred culture media include DMEM (Dulbecco's modified essential medium) and NPBM (Neural progenitor cell basal medium; Clonetics). There is no limitation on the types of other constituents that may be contained in the above-mentioned basal culture medium, and preferred constituents include F-12, FCS and Neural survival factors (Clonetics). In such culture media, for example, the concentrations of F-12 and FCS are 50% and 1%, respectively. The CO₂ concentration in such a culture media is preferably 5% but the invention is not so limited thereto.

According to the present invention, the differentiation of the above-mentioned mesodermal stem cells into the above-mentioned neural cells, including neural stem cells, can be promoted by adding ischemic brain extract to the above-mentioned basal culture medium or the basal culture medium containing other constituents. Generally, in the absence of such extracts of ischemic brain tissue, the differentiation into neural stem cells takes one week or more. However, when such an extract is added, the induction can be achieved in only several days and furthermore, the differentiation efficiency is highly improved. The present invention also provides such a method for promoting the induction of differentiation into neural cells, including neural stem cells.

The ischemic brain extract of the present invention can be prepared, for example, by centrifuging crushed lysate of ischemic brain from a vertebrate animal. Specifically, the whole brain is excised from a whole brain ischemia model animal (rat, etc.), small pieces are prepared from the brain and combined with NPBM (culture medium for neural stem cells). The small pieces are mechanically crushed in a homogenizer. Then, the sample is centrifuged at 300 g for 5 minutes or at 800 rpm for 5 minutes, the resulting supernatant is collected, and the supernatant is filtered through a membrane filter to remove cell debris to prepare an extract of ischemic brain. However, the present invention is not restricted to this method. An animal for the whole brain ischemia model can be prepared by anesthetizing an animal with Nembutal and then perfusing the animal with physiological saline. The extract of ischemic brain, prepared by the procedure described above is then added to the above-mentioned basal culture medium or the basal culture medium containing other components. Again, there is no limitation on the timing of addition.

According to the present invention, the above-mentioned mesodermal stem cells are differentiated into neural cells by culturing the cells under the condition as described above. Specific examples of resulting neural cells include: neural stem cell, neural progenitor cell, neuron, and glial cell.

The culture temperature used in the method of the present invention falls within the range of 33°C to 38°C, and a preferred temperature is 37°C.

There is no limitation on other parameters of culture conditions. The cells may be in the state of suspension (neurosphere state) or adhered to the culture container. Suitable culture containers include, for example, non-coated dishes and such.

Furthermore, the safety of the neural cells whose differentiation is induced by the method of the present invention can be improved through the removal of the immortalization gene. In the present invention, the immortalization gene introduced into a cell can be removed from the cell using established techniques. The immortalization gene can be specifically removed via treatment with a recombinase, such as the Cre recombinase, for example, by placing the immortalization gene between a pair of loxP sequences or loxP-like sequences.

According to the present invention, the mesodermal stem cells can also be differentiated directly into neurons or glial cells (without conversion to neural stem cells) by changing the culture medium of the above-mentioned mesodermal stem cells with a fresh culture medium [50% DMEM (Dulbecco's modified essential medium)/50% F-12/1% FSC] or culture medium 2 [NPBM (Neural progenitor cell basal medium; Clonetics)/2% Neural survival factors (Clonetics)/0.2% Gentamicin-amphotericin B/10 ng/ml hFGF] and further culturing for about several days to about 4 weeks. Thus, the present invention also includes the method for inducing differentiation of mesodermal stem cells directly into neuron or glial cells.

The present disclosure further provides cells prepared by the above-mentioned method of the present invention. Neural cells are provided by the present disclosure and include, but are not limited to, neural stem cell, neural progenitor cell, neuron, and glial cell.

The present disclosure also provides a composition for treating neurological diseases, which composition comprises cells that prepared by the above-mentioned method. The cells of the present disclosure may be directly used for transplantation. However, to improve therapeutic efficiency, the cells may be transplanted as a composition supplemented with various agents or a composition wherein genes are introduced.

When intending to prepare the composition of the present disclosure, for example, one can: <1> add a substance that improves the proliferation rate of cells of the present invention or that promotes the differentiation into neural cells, or introduce a gene that directs such effects; <2> add a substance that improves the viability of cells of the present invention in damaged neural tissues, or introduce a gene that directs the same effect; <3> add a substance that inhibits adverse influences from damaged neural tissues to the cells of the present invention, or introduce a gene that directs the same effect; <4> add a substance that prolongs the life span of donor cells, or introduce a gene that directs the same effect; <5> add a substance that regulates the cell cycle, or introduce a gene that directs the same effect; <6> add a substance that is used to suppress the immune reaction, or introduce a gene that directs the same effect; <7> add a substance that activates the energy metabolism, or introduce a gene that directs the same effect; <8> add a substance that improves the migration activity of donor cells in host tissues, or introduce a gene that directs the same effect; <9> add a substance that improves blood flow, or introduce a gene that directs the same effect (including induction of angiogenesis); and <10> add a substance that has a certain therapeutic effect on host brain and/or nerve (with diseases to be treated including, for example, brain tumor, demyelination diseases in the central and peripheral nervous systems, degenerative diseases in the central and peripheral nervous systems, brain apoplexy (including brain infarction, brain hemorrhage and subarachnoid hemorrhage), higher brain dysfunction including dementia, psychiatric diseases, epilepsy, traumatic neurological diseases (including head injury, brain contusion and spinal cord injury), inflammatory diseases, infectious diseases, etc.), or introduce a gene that directs the same effect. However, the present invention is not limited to these examples.

The cells and compositions of the present disclosure can be transplanted into recipients for the purpose of treating neurological diseases. The neurological diseases to be treated include, but are not limited to, demyelination diseases in the central and peripheral nervous systems, degenerative diseases in the central and peripheral nervous systems, brain apoplexy (including brain infarction, brain hemorrhage and subarachnoid hemorrhage), brain tumor, higher brain dysfunction including dementia, psychiatric diseases, epilepsy, traumatic neurological diseases (including head injury, brain contusion and spinal cord injury), inflammatory diseases, infectious diseases (for example, Creutzfeldt-Jakob disease) and infarction of spinal cord.

According to the present disclosure, recipient-derived cells that are isolated, for example, from bone marrow fluid, umbilical blood, peripheral blood, skin, muscle tissue, fat tissue, synovial membrane and hair root can be transplanted as donor cells (autotransplantation therapy). Autotransplantation therapy is preferable due to its low risk of rejection and avoidance of a need for immunosuppressants. In case where autotransplantation therapy is difficult, cells of other human or animals bred for medical purposes may be used.

The present inventors also examined the relationship between the timing of cell transplantation and the degree of therapeutic effect, and found out that the highest therapeutic effect can be obtained when the transplantation therapy was conducted in the hyperacute phase (within several hours). Second to the therapy conducted in the hyperacute phase, a high therapeutic effect can be obtained by conducting the therapy in the acute phase (after several days), and third by conducting the therapy in the chronic phase (after several weeks). Therefore, the transplantation therapy in the hyperacute phase is considered preferable. Thus, it is exceedingly useful to previously collect and culture cells, introduce genes into the cells, proliferate the cells, induce differentiation of the cells, and store the cells. Specifically, it is preferable to previously collect cells from a recipient, introduce an immortalization gene into the cells according to the method of the present invention, and induce differentiation into neural cells by cultivation to appropriately store the cells for transplantation. The cells may be stored at any state listed below. One skilled in the art can appropriately select a proper state of the cells considering the type of the disease to be treated from:
1. cells collected without any treatment (crude fraction);
2. partially purified cells;
3. cells purified and then proliferated by cultivation;
4. cells purified, immortalized, and proliferated;
5. cells differentiated into neural stem cells; and
6. cells differentiated into neural cells
Furthermore, cells registered in bone marrow banks, cord blood banks and the like can also be used for transplantation. Such cells may be stored frozen.

In addition, the present disclosure revealed that, when mesodermal stem cells (including immortalized cells) are transplanted into a recipient without any further treatment, the mesodermal stem cells migrated into neural tissues and differentiated into neural cells to result in functional regeneration. Accordingly, the method of directly administering (transplanting) mesodermal stem cells to a recipient and inducing differentiation of the cells into neural cells *in vivo* is also encompassed in the present disclosure. There is no limitation on the type of administration procedure to be used in this method. Suitable administration procedures include, for example, local administration, intravenous administration, intraarterial administration and intra-cerebrospinal administration (for example, lumbar puncture and intraventricular administration).

Cell transplantation into patients can be achieved, for example, by filling a syringe with cells to be transplanted, suspended in artificial cerebrospinal fluid, physiological saline or the like, exposing damaged neural tissue by operation, and directly injecting the cells into the damaged area with an injection needle. The cells of the present disclosure can then migrate into neural tissues due to their high migration activity. Therefore, the cells may be transplanted into a portion adjacent to a damaged area. Injection of the cells into the cerebrospinal fluid may also be effective. In this case, the cells can be injected by typical lumbar puncture, and thus is preferable, since the patient is treated with only local anesthesia and without operation in a sickroom. Moreover, intraarterial injection and intravenous injection can also be effective, and hence the transplantation can be practiced by the same procedure as typical blood transfusion. Such methods are preferable to those transplantation methods that require the use of a sickroom.

In addition, due to their high migration activity, the cells of the present disclosure can be used as a carrier (vector) for genes. The cells can be used as vectors for gene therapy for various neurological diseases, for example, brain tumor, demyelination diseases in the central and peripheral nervous systems, degenerative diseases in the central and peripheral nervous systems, brain apoplexy (including brain infarction, brain hemorrhage and subarachnoid hemorrhage), higher brain dysfunction including dementia, psychiatric diseases, epilepsy, traumatic neurological diseases (including head injury, brain contusion and spinal cord injury), inflammatory diseases and infectious diseases '(for example, Creutzfeldt-Jakob disease).

### Brief Description of the Drawings

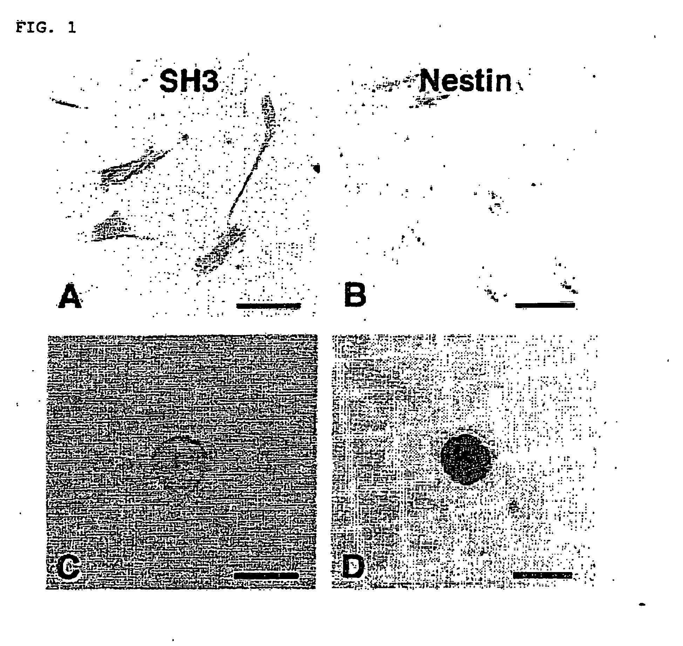

Fig. 1 shows photographs depicting cultured mesodermal stem cells. The cells express the mesodermal cell marker SH3 (A), but are negative to the neural stem cell marker nestin (B). After the induction of differentiation under the culture condition, the cells morphologically changed to a neural stem cell-like shape and became negative to the mesodermal cell marker SH3 (C) and positive to nestin (the neural stem cell marker) (D). Scale bars indicate 10 µm in (A) and (B), and 200 µm in (C) and (D).

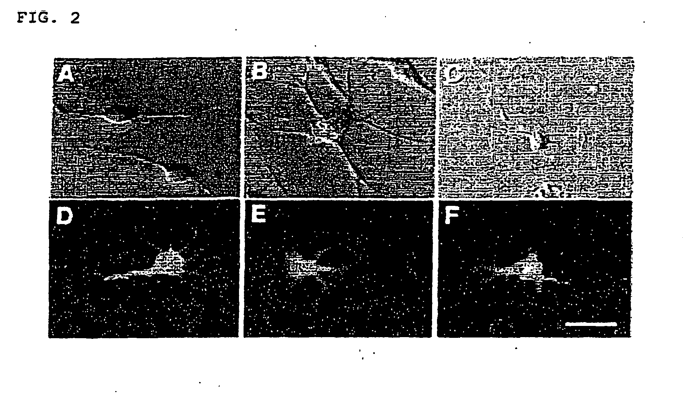

Fig. 2 shows photographs depicting the differentiation of neural stem cells that were differentiated from mesodermal stem cells under the culture condition into neuron (A, D), astrocytes (B, E), and oligodendrocytes (C, F) upon further induction. The cells were immuno-stained with NSE (neuron-specific enolase) (D); with GFAP (glial fibrillary acidic protein) (E); and with GalC (galactocerebroside) (F). Scale bars indicate 25 µm.

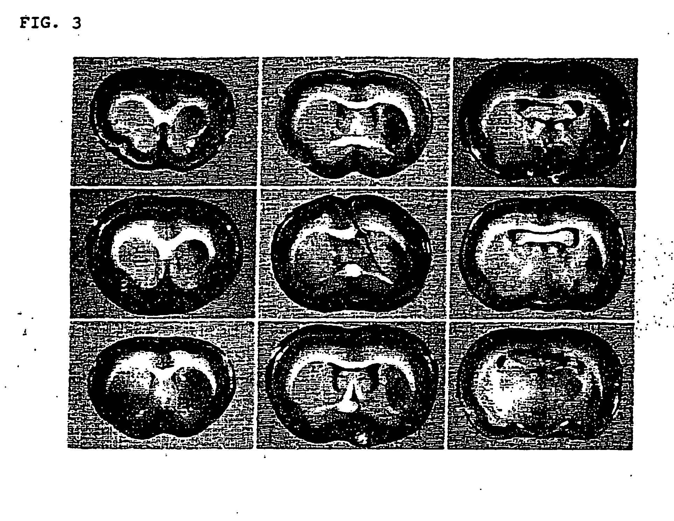

Fig. 3 shows photographs depicting the repair of the lesions of brain infarction by the transplanted cells. The donor cells, i.e., neural stem cells whose differentiation was induced from mesodermal stem cells under the culture condition, were genetically labeled with the LacZ gene (expressing the *E. coli* β-galactosidase). Hence, the cells are stained blue when treated with the substrate X-gal. This enables the tracing of the donor cells in tissues of the host brain. As a result of transplantation of the donor cells labeled with the LacZ gene into a rat brain infarction model (temporary middle cerebral artery infarction model; brain infarction occurs in cerebrum basal ganglia, temporal lobe, hippocampus, etc.), the cells take to cerebrum basal ganglia, temporal lobe, hippocampus, etc., affected with brain infarction to repair the tissues.

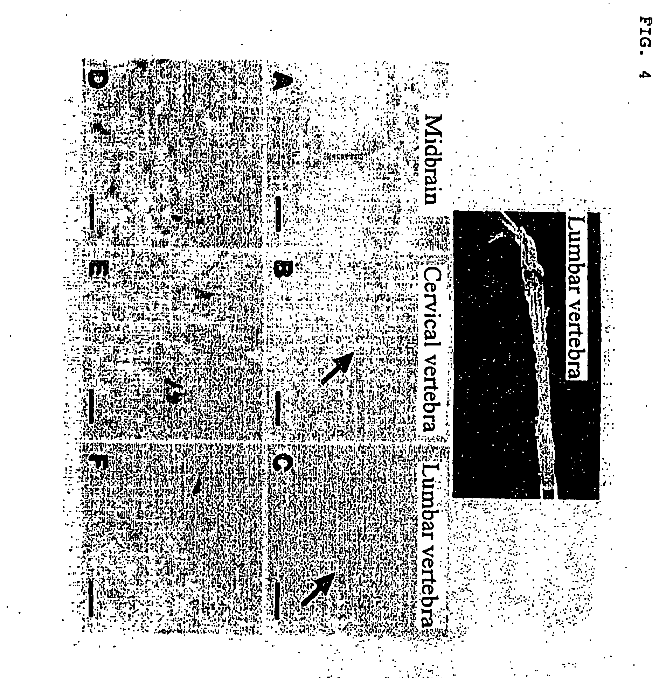

Fig. 4 shows photographs depicting the repair of damaged areas of the spinal cord by the transplanted cells. As a result of transplantation of the donor cells labeled with the LacZ gene to a rat spinal cord injury model (a model wherein the spinal is cut at the first thoracic level), the donor cells migrated not only into the damaged area but also to the brain (A), cervical spinal cord (B) and lumbar cord (C), and repaired the tissues. The photographs (D), (E), and (F) correspond to photographs (A), (B), and (C), respectively, observed with higher magnification. Scale bars indicate 200 µm in (A)-(C), and 10 µm in (D)-(F).

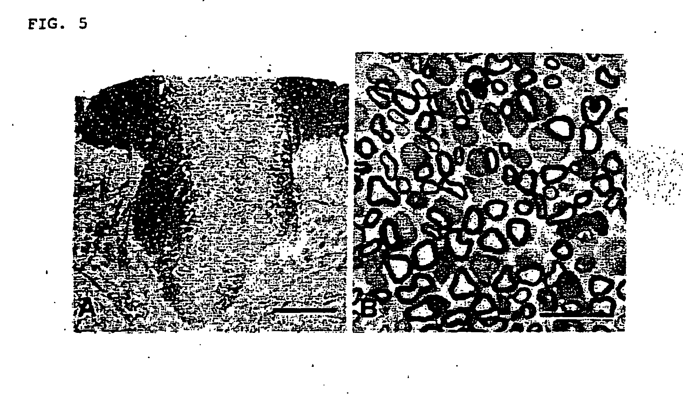

Fig. 5 shows photographs depicting the repair of demyelinated areas of the spinal cord by the transplanted cells. (A) is a photograph showing the re-myelination after transplantation of neural stem cells into the demyelinated area of the spinal cord of a mature rat. The neural stem cells had been differentiated from human mesodermal stem cells obtained from an adult. (B) is a photograph showing the re-myelinated axons observed with higher magnification.

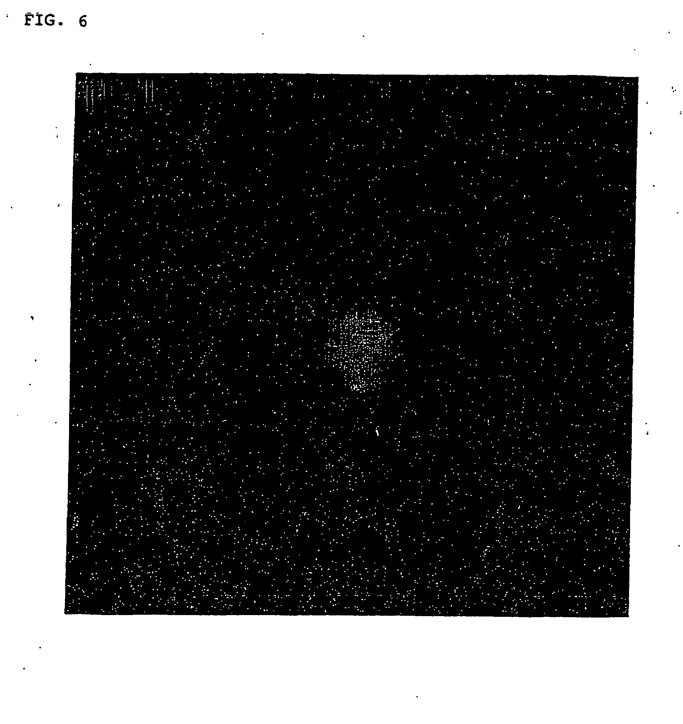

Fig. 6 shows a photograph depicting the nestin-positive neurosphere of a neural stem cell differentiated from an ES cell.

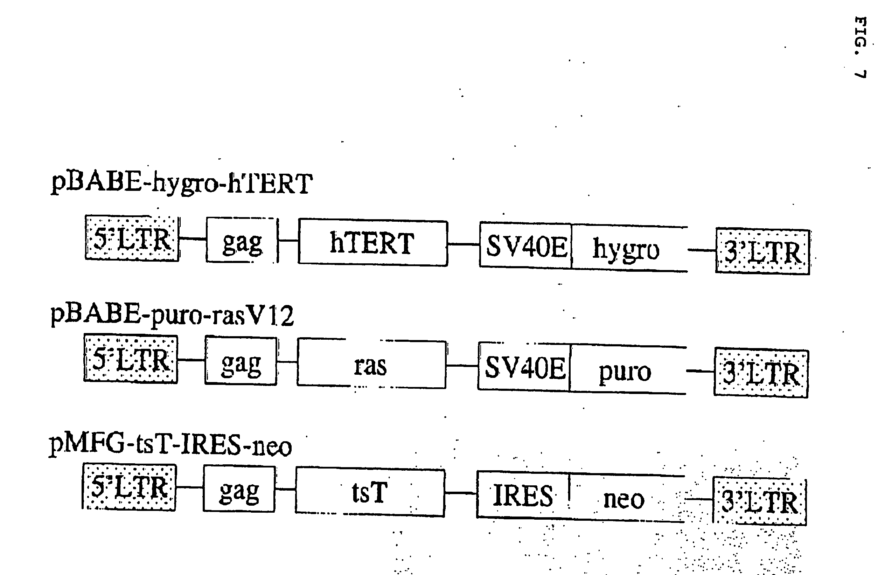

Fig. 7 shows schematic illustrations of the structure of vectors used for gene introduction into stromal cells.

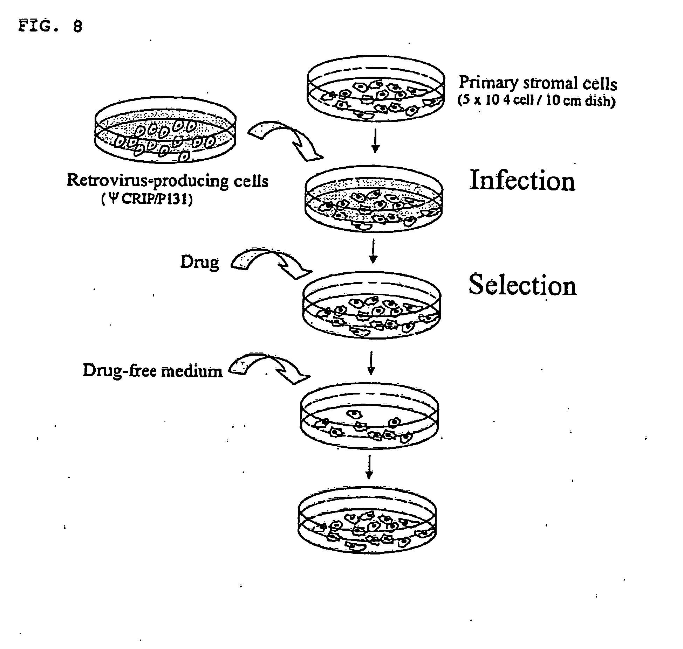

Fig. 8 shows a schematic illustration of transfection into stromal cells.

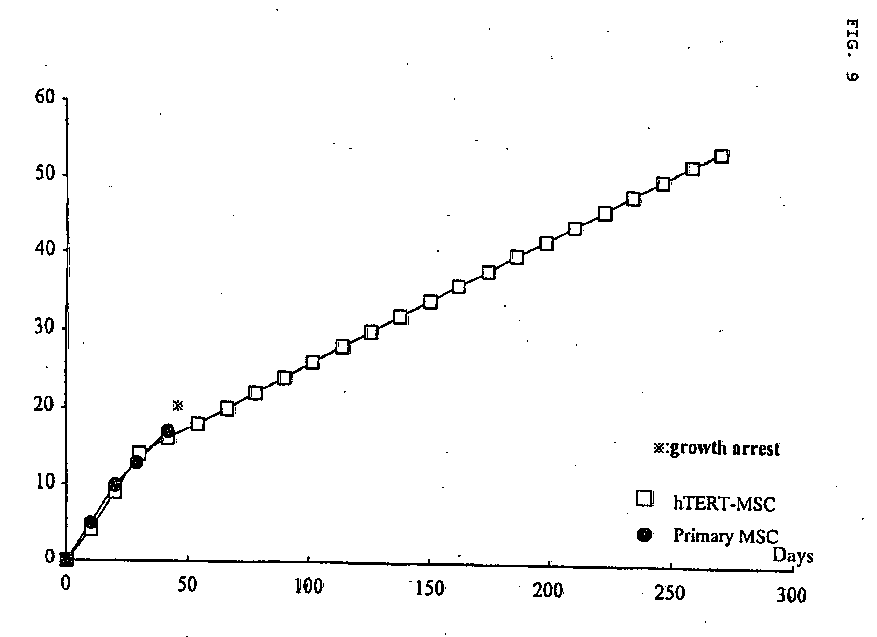

Fig. 9 shows the numbers of generations with respect to cell division of mesodermal stem cells (primary culture) and mesodermal stem cells immortalized by introducing hTERT.

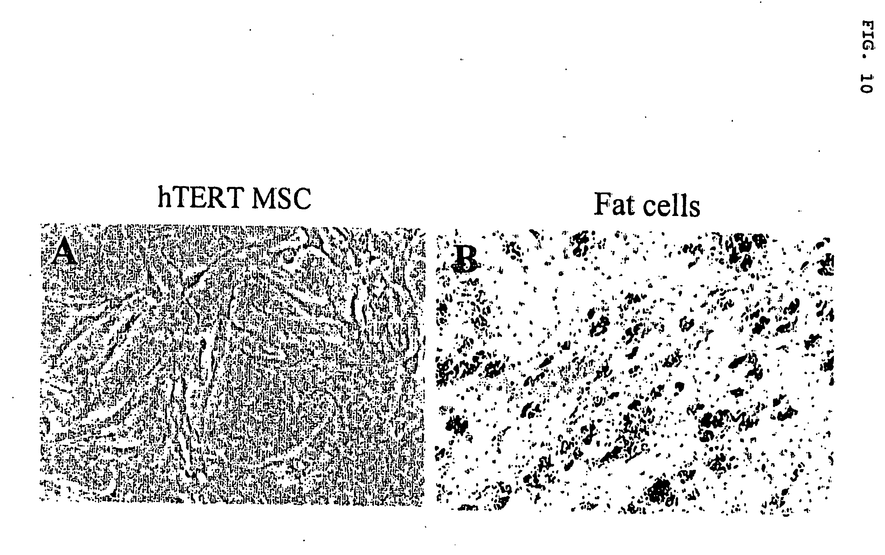

Fig. 10 shows photographs depicting mesodermal stem cells immortalized by introducing hTERT (A) and fat cells (B: Oil Red O-staining) differentiated from the immortalized mesodermal stem cells.

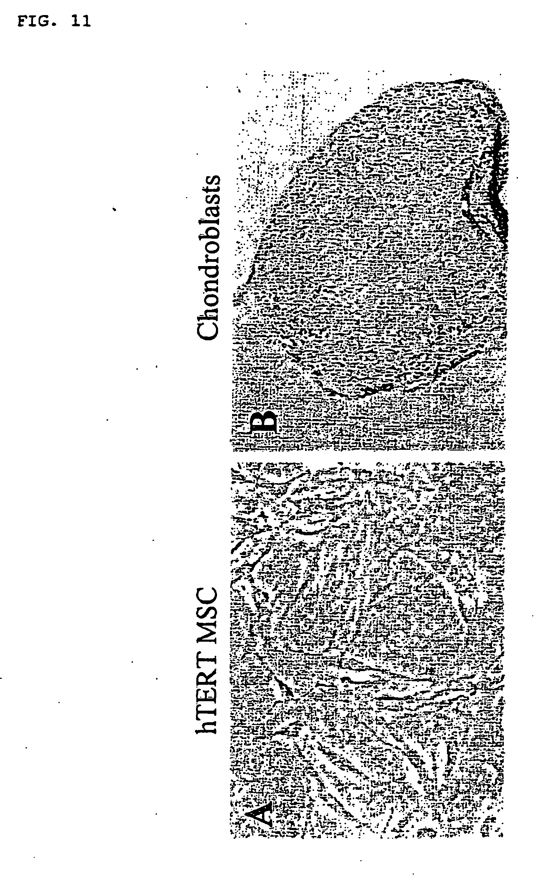

Fig. 11 shows photographs depicting mesodermal stem cells immortalized through the introduction of hTERT (A) and chondroblasts (B: Alcian blue-staining) differentiated from the immortalized mesodermal stem cells. The chondroitin in the cartilage matrix (frozen section) was stained blue.

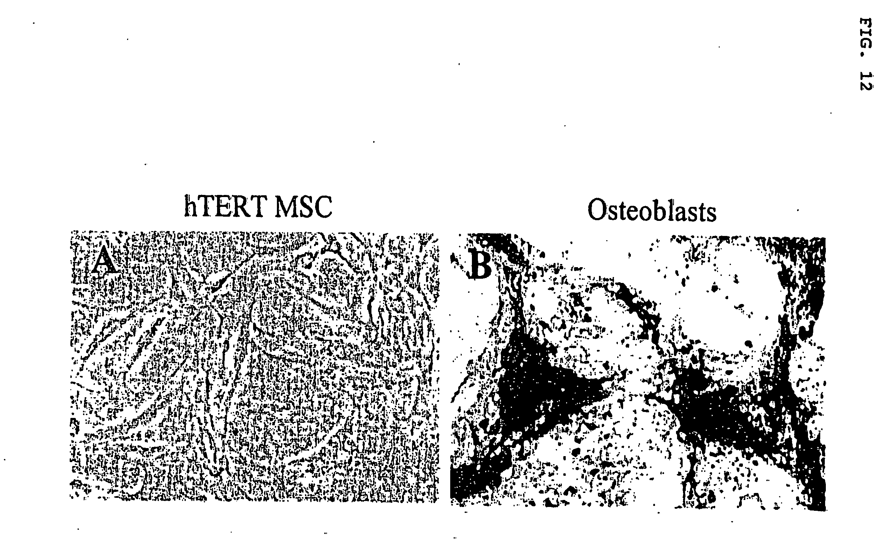

Fig. 12 shows photographs depicting mesodermal stem cells immortalized through the introduction of hTERT (A) and osteoblasts (B: von Kossa staining; deposition of minerals) differentiated from the immortalized mesodermal stem cells.

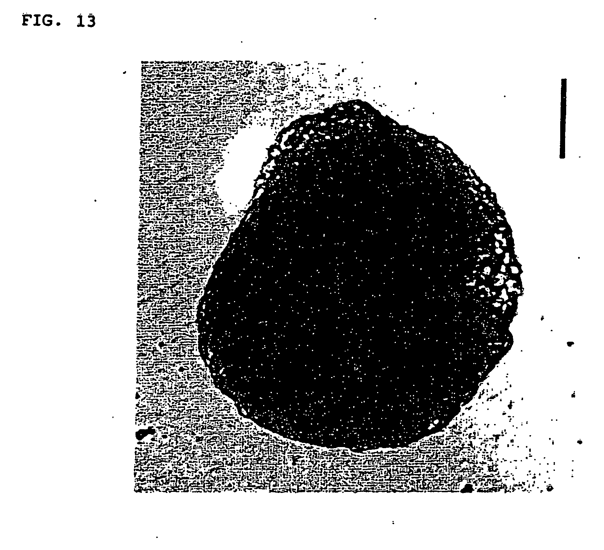

Fig. 13 shows a photograph depicting nestin-positive neural stem cells differentiated from mesodermal stem cells immortalized through the introduction of hTERT. The scale bar indicates 100 µm.

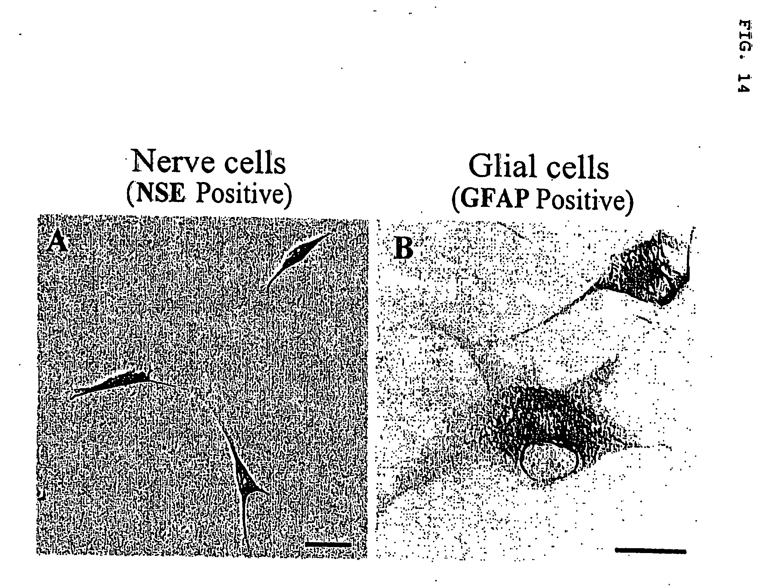

Fig. 14 shows photographs depicting NSE-positive neurons (A) and GFAP-positive glial cells (B), both differentiated from neural stem cells that were differentiated from mesodermal stem cells immortalized through the introduction of hTERT. Scale bars indicate 20 µm in (A) and 10 µm in (B).

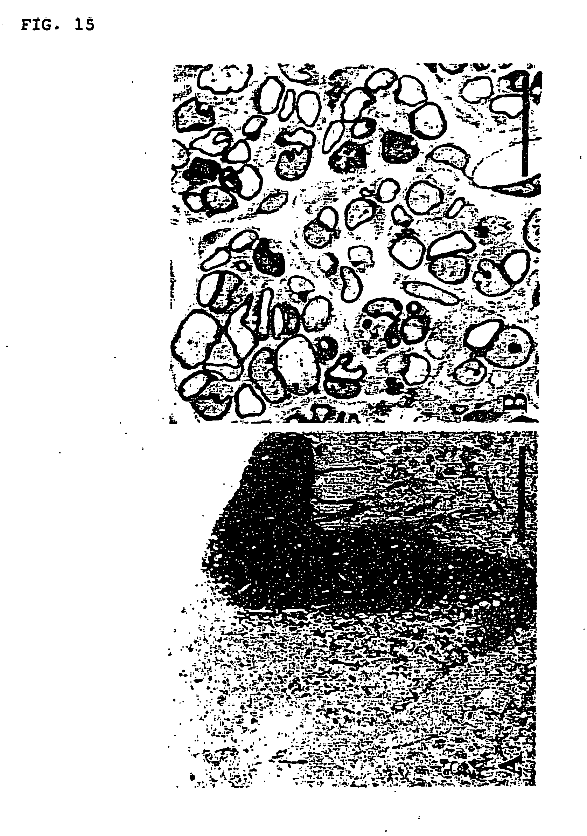

Fig. 15 shows photographs depicting the repair of demyelinated areas in the spinal cord by the transplantation of mesodermal stem cells immortalized through the introduction of hTERT. (A) is a photograph showing the re-myelination of demyelinated area in the spinal cord of a mature rat after transplantation of mesodermal stem cells introduced with the hTERT gene. (B) is a photograph showing the re-myelinated axons observed with higher magnification. Scale bars indicate 250 µm in (A) and 10 µm in (B)

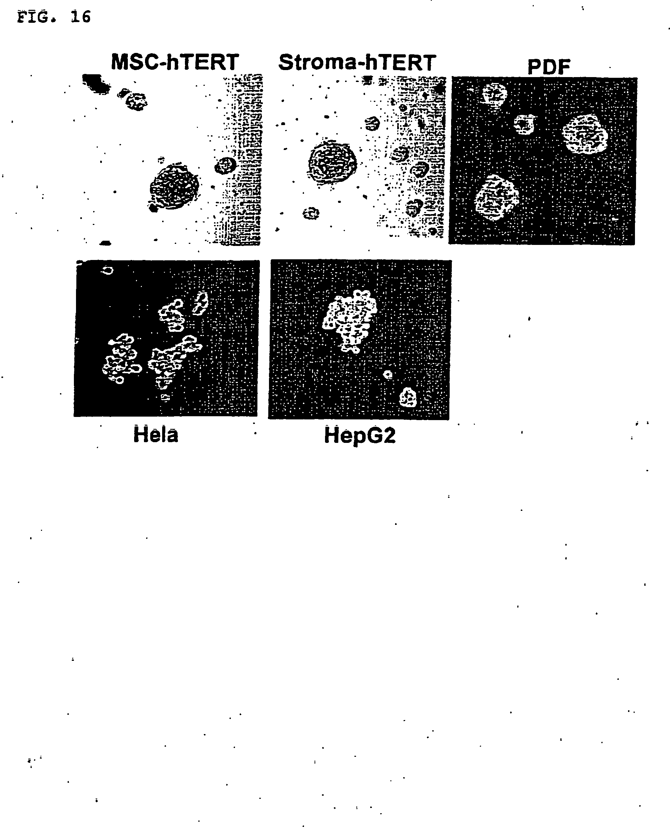

Fig. 16 shows photographs depicting the results obtained by inducing differentiation of the respective cells into neural stem cells according to the method of the present invention. The respective cells were incubated in suspension state in Neural Progenitor Basal medium on non-treated dishes. The cells were observed after 48 hours. All cells were viable in the suspension state. Only MSC-hTERT, Stroma-hTERT, and PDF showed a neurosphere-like morphology.

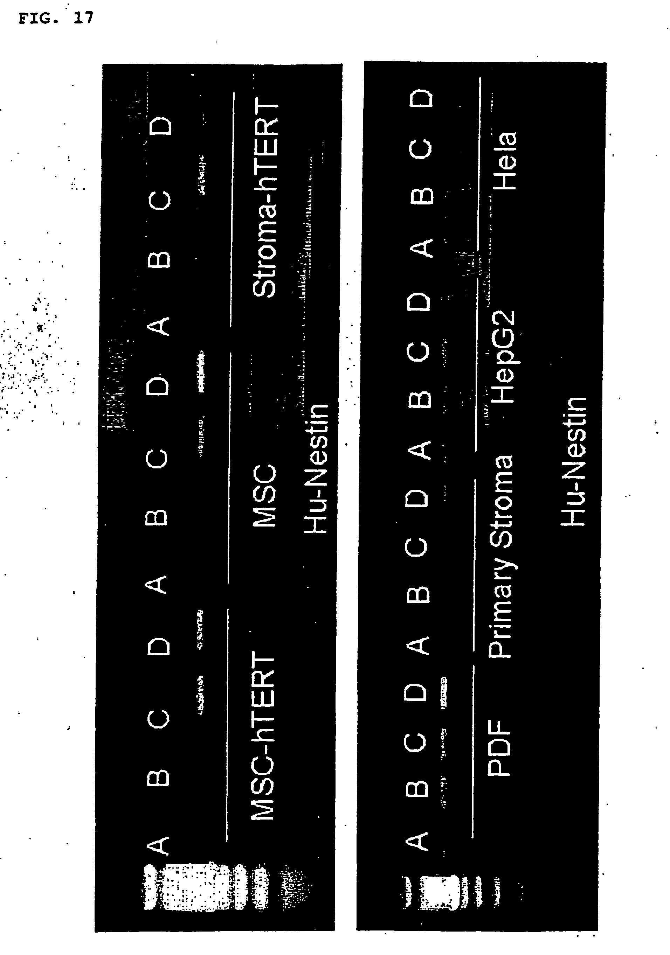

Fig. 17 shows photographs depicting the results of an RT-PCR assay confirming the expression of nestin using total RNAs prepared from the respective cells after the induction treatment for differentiation into neural stem cell using the method of the present invention. Lane A corresponds to DMEM-10% FBS/culture dish; B, NPBM/non-treated dish/1 day; C, NPBM/non-treated dish/2 days; and D, NPBM/non-treated dish/5 days. NPBM refers to Neural Progenitor basal medium.

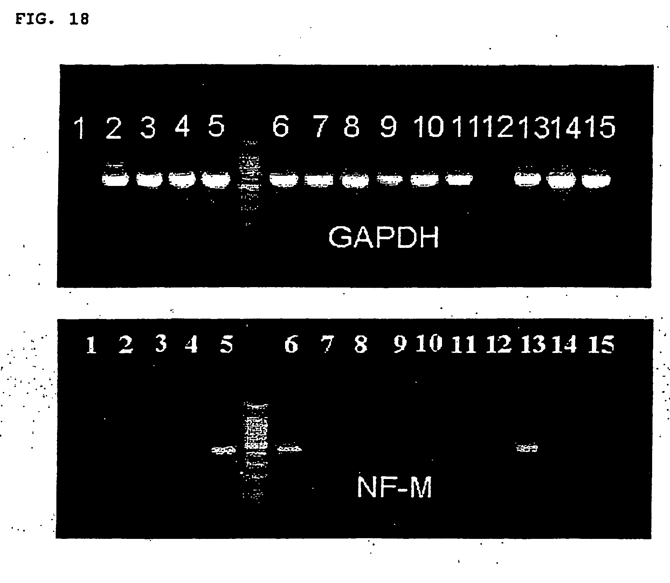

Fig. 18 shows photographs depicting the results of RT-PCR assay confirming the differentiation into neurons of neural stem cells differentiated from MSC. The top panel shows the result normalized with GAPDH (GAPDH was used as an internal control); the bottom panel shows the results normalized with a subunit of neurofilament (NF), NF-M. Lane 1 corresponds to DDW; lane 2, neural stem cell; lane 3, MSC-hTERT; lane 4, Stroma-hTERT; lane 5, PDF-hTERT; lane 6, MSC-hTERT BHA, DMSO; lane 7, Stroma-hTERT BHA, DMSO; lane 8, PDF-hTERT BHA, DMSO; lane 9, MSC-hTERT NPBM(-); lane 10, Stroma-hTERT NPBM(-); lane 11, PDF-hTERT NPBM(-); lane 12, NSC NPBM(-) PDL/laminin; lane 13, MSC-hTERT NPBM(-) PDL/laminin; lane 14, Stroma-hTERT NPBM(-) PDL/laminin; and lane 15, PDF-hTERT NPBM(-) PDL/laminin.

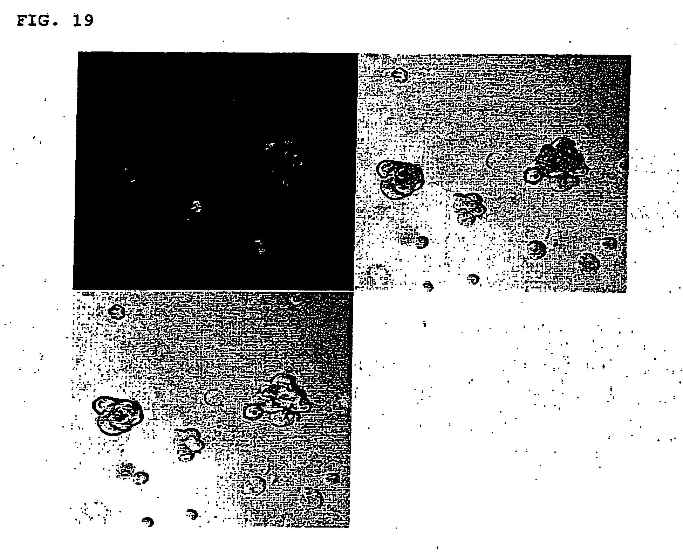

Fig. 19 shows photographs depicting the results of induction of differentiation into neural stem cells of MSC-hTERT introduced with a vector according to the method of the present invention. The vector expresses EGFP in nestin positive cells. The cells were observed under a confocal laser microscope. As a result, MSC-hTERT was revealed to express no EGFP before the differentiation into neural stem cells but strongly express EGFP after the induction.

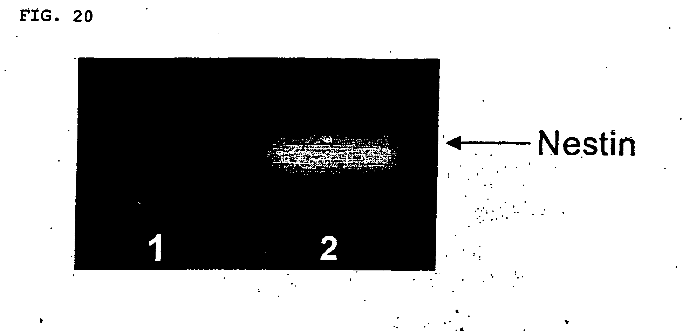

Fig. 20 shows a photograph depicting the results of an RT-PCR assay confirming nestin expression in MSC-hTERT. Lane 1, cells of MSC-HTERT containing introduced nestin enhancer/promoter EGFP; and lane 2, cells of MSC-hTERT introduced with nestin enhancer/promoter EGFP and then differentiated into neural stem cells.

### Best Mode for Carrying out the Invention

The present invention is illustrated in detail below with reference to the Examples below, but should not be construed as being limited thereto.

### Example 1: Preparation of mononuclear cell fraction and mesodermal stem cells

### (1) Mononuclear cell fraction and culture solution of the mononuclear cell fraction:

A cell sample collected from mouse (or human) was diluted with L-15 medium (2 ml) containing 3 ml of Ficoll, and centrifuged at 2,000 rpm for 15 minutes. Cells were isolated from the mononuclear cell fraction, and suspended in 2 ml of serum-free medium (Neural Progenitor cell Maintenance Medium (NPMM)). The suspension was centrifuged at 2,000 rpm for 15 minutes. The supernatant was discarded and the precipitated cells were collected. The cells were resuspended in NPMM to prepare a mononuclear cell fraction.

Further, culture solutions of the mononuclear cell fraction were prepared by incubating the mononuclear cell fraction at 37°C under 5% CO₂ in culture medium 1 [DMEM (Dulbecco's Modified Eagles Medium-Low Glucose)/10% FBS (fetal bovine serum)1% anti-biotic-antimycotic solution], culture medium 2 [MSCBM (Mesenchymal Stem Cell Basal Medium)/10% MCGS (Mesenchymal Cell Growth Supplement)/4 mM L-glutamine/1% penicillin-streptomycin], and culture medium 3, respectively,

### (2) Mesodermal stem cell:

Using antibodies, cells having the characteristics of SH2(+), SH3(+), SH4(+), CD29(+), CD44(+), CD14(-), CD34(-), CD45(-), and such were selected from the mononuclear cell fraction or the culture solution of the mononuclear cell fraction prepared by the procedure described above in (1). The selection was conducted by a method using magnetic beads or a typical cell sorter (FACS, etc.).

### Example 2: Induction of differentiation of mesodermal stem cells into neural cells

### (1) Induction of differentiation of mesodermal stem cells into neural stem cells:

First, the cells were washed and then the cells in the culture medium were collected upon enzymatic treatment (reagents: 0.05% trypsin/0.02% EDTA; at room temperature for 5 minutes). After adding an equal volume of culture medium, the cells were dispersed into single cells by pipetting for several times. Then, the cells were centrifuged at 600 rpm for 5 minutes. The precipitated cells were sucked up with a pipette. Then, the cells were further cultured in a floating state at 37°C under 5% CO₂ in fresh culture medium 1 [50% DMEM (Dulbecco's modified essential medium)/50% F-12/1% FSC; 10 ng/ml basic fibroblast growth factor (bFGF) added every day; 10 ng/ml epidermal growth factor (EGF) added every day] or culture medium 2 [NPBM (Neural progenitor cell basal medium; Clonetics)/2% Neural survival factors (Clonetics)/0.2% hEGF (human epidermal growth factor)/0.2% Gentamicin-amphotericin B/0.2% hFGF (human fibroblast growth factor); 10 ng/ml basic fibroblast growth factor (bFGF) added every day; 10 ng/ml Epidermal growth factor (EGF) added every day] in a culture container (non-treated polystyrene dish).

### (2) Induction of differentiation of mesodermal stem cells into neurons or glial cells:

The culture medium of mesodermal stem cells under culture was changed with a fresh culture medium [50% DMEM (Dulbecco's modified essential medium)/50% F-12/1% FSC] or culture medium 2 [NPBM (Neural progenitor cell basal medium: Clonetics)/2% Neural survival factors (Clonetics)/0.2% hEGF (human epidermal growth factor)/0.2% Gentamicin-amphotericin B/0.2% hFGF]. Direct differentiation of mesodermal stem cells into neurons or glial cells (without conversion into neural stem cells) was successfully achieved by culturing for about 4 weeks.

The induction of differentiation of mesodermal stem cell into neural cells using the above-mentioned method (1) or (2) was determined by confirming the disappearance of mesodermal cell markers SH2 and SH3 (the cells were originally negative to the neural stem cell marker nestin) and the appearance of the neural stem cell marker nestin (Fig. 1).

In addition, the cultured mesodermal stem cells morphologically changed upon differentiation into neural cells. Specifically, the dispersed cells formed a neurosphere (Fig. 1). Furthermore, these neural stem cells were confirmed to differentiate into neurons (NSE positive) and glial cells (GFAP positive) (Fig. 2).

### Example 3: Promotion of induction of differentiation of mesodermal stem cells into neural cells using ischemic brain extract

Mesodermal stem cells were revealed to differentiate into neural stem cells with higher efficiency by adding ischemic brain extract, prepared from brain exposed to ischemic stress, during the culture of mesodermal stem cells. This method was revealed to ensure highly efficient differentiation of mesodermal stem cells into neural stem cells in only several days of culture.

### (1) Preparation of ischemic brain extract:

First, a rat was deeply anesthetized with Nembutal and the precordial region was dissected from the anterior abdominal wall to expose the heart and ascending aorta. The apex cordis was incised and a tube was inserted from the left ventricle to the ascending aorta. Then, an incision was made in the right auricle of heart, and physiological saline was perfused from the tube for 3 minutes for sufficient general blood letting. After the perfusion, the rat was left still for 4-5 hours and used as a whole brain ischemia model. Next, the cerebrum and midbrain were resected from the above-mentioned whole brain ischemia model rat and cut into small sections of about 1-2 mm with a spitstick. The prepared small sections were combined with NPMM and mechanically crushed in a homogenizer to prepare a suspension. Then, the suspension was transferred into a centrifugation tube, centrifuged at 800 rpm for 5 minutes, and the resulting supernatant was collected. Cell components were removed with a 0.22-µm membrane filter and the filtrate was used as ischemic brain extract. .

### (2) Induction of differentiation of cultured mesodermal stem cell (MSC) into neural stem cells:

MSCs were further cultured in conditioned media (3 types: described herein elsewhere) on a 100-mm non-coated dish (IWAKI) at 37°C under 5% CO₂ until 90% confluence. Next, the culture liquid was sucked up with a Pasteur pipette and the cells were washed three times with Dulbecco's PBS. 2 ml of PBS containing 0.05% trypsin and 0.02% EDTA was added to the dish and then incubated at 37°C for 2-5 minutes until the cells detached. 2 ml of the conditioned medium was added to the dish to quench trypsin reaction. The supernatant and detached cells were transferred into a centrifugation tube with a Pasteur pipette, pipetted for, several times and then centrifuged at 1000 rpm for 5 minutes. The supernatant was discarded, and the cells were resuspended in NPMM. The cells were plated on a medium (50% NPMM and 50% ischemic brain extract) pre-warmed at 37°C, and then cultured in a suspension state in a 100-mm non-coating dish (IWAKI) at 37°C under 5% CO₂. 10 ng/ml bFGF and 10 ng/ml EGF were added every day.

### Example 4: Evaluation of neural regeneration potency

The neural regeneration potency of neural cells obtained by the above-described method for inducing differentiation was assessed using a brain infarction model (Fig. 3), a dementia model (Fig. 3), a spinal cord injury model (Fig. 4) and a demyelination model (Fig. 5). As a result, the cells were determined to have regeneration potency comparable to that of neural stem cells extracted and cultured from the brain.

### Example 5: Introduction of hTERT gene into cells

A method for immortalizing stromal cells by introducing hTERT gene into the cells is described below:
1. Collection of primary stromal cells:
   Mononuclear cells were isolated from 10 ml of bone marrow fluid obtained from the iliac bone of a healthy adult male human. Cells adhering to the flask after overnight culture were used as stromal cells.
2. A gene encoding the catalytic subunit of human telomerase (hTERT) was introduced into stromal cells. The sequence of hTERT is shown, for example, in Science 277: 955-959. The ras gene and the SV40 T gene, both known as genes associated with cell oncogenesis, were also introduced into the stromal cells.
3. Vector pBABE-hygro-hTERT (gift from Dr. Robert A. Weinberg; Fig. 7) used for gene introduction into stromal cells was constructed by cloning, into pBABE-hygro, hTERT *EcoR*V*-sal*I fragment obtained from pCI-Neo-hTERT-HA by PCR as described in Proc. Natl. Acad. Sci. USA 95: 14723-14728. Vector pBABE-puro-rasV12 (gift from Dr. Scott W Lowe) was prepared according to the method described in Cell, 88: 593-602, 1997. Vector pMFG-tsT-IRES-neo was prepared by cloning the *Bam*HI fragment of IRES-neo (obtained by digesting pRx-hCD25-ires-neo (Human gene therapy 9: 1983-1993, 1998)) into MFG-tsT (prepared by inserting a fragment of pZIPtsU19 (gift from Dr. R. McKay) into MFG vector
   (Lab. Invest. 78: 1467-1468, 1998)).
4. Preparation of retrovirus-producing cells and virus infection using the cells was carried out according to the method described in "Experimental Medicine, Supplement: The Protocol Series - Experimental Methods for Gene Delivery & Expression Analysis (Eds., 1. Saito and S. Sugano; Yodosha)" (pp. 58-62).

Specifically, ΨCRIP packaging cells (Proc. Natl. Acad. Sci. USA, 90: 3539-3543, 1993) were prepared using BOSC23 packaging cells (Proc. Natl. Acad. Sci. USA, 90: 8392-8396, 1993) by the procedure described below.

### 4-1. Preparation of cells producing recombinant retroviral vector:

i) BOSC23 cells were plated on a 10-cm dish at a cell density of 5.5x10⁶ cells/dish 18-24 hours before transfection.
ii) Solution A was prepared by gently adding 800 µl of OPTI-MEM (Gibco/BRL) to 15 µg DNA (retroviral vector) and stirring the mixture.
iii) Solution B was prepared by placing 750 µl of OPTI-MEM into a sterilized tube, adding 50 µl of LIPOFECTAMINE (2 mg/ml Gibco/BRL) thereto and gently agitating the mixture.
iv) Solution C was prepared by gently mixing Solution B into Solution A, and then leaving at room temperature for 30-45 minutes.
v) BOSC23 cells were washed once with antibiotic-free, FBS-free medium pre-warmed at 37°C.
vi) Solution C (1.6 ml) was added gently to the BOSC23 cells.
vii) 2.4 ml of OPTI-MEM was also added to the mixture.
viii) The resulting mixture was incubated for 5 hours under 5% CO₂.
ix) 4 ml of DMEM containing 20% fetal bovine serum was added and the mixture was incubated overnight.
x) The medium was changed with fresh medium containing 10% fetal bovine serum pre-warmed at 37°C, and at the same time packaging cells ΨCRIP were plated on a 10-cm dish at a cell density of 1-2x 10⁶ cells/dish.
xi) After 24 hours, the medium of BOSC23 cells was filtered through a 0.45- or 0.20-µm syringe filter. The medium of ΨCRIP was changed with 5 ml of the filtered medium. At the same time, Polybrene (Hexadimethrine Bromide, SIGMA H-9268) was added at a final concentration of 8 µg/ml.
xii) After 4 to 24-hours culture, 5 ml of medium was added, and the cells were further incubated overnight.
xiii) Finally, the retrovirus-producing ΨCRIP cells were prepared by drug selection.

Then, the above-mentioned three types of vectors were separately proliferated in retrovirus-producing cells (ΨCRIP/P131). The stromal cells were transfected as follows (Fig. 8).

First, one day before transfection, the stromal cells were re-plated at a cell density of 5x 10⁴ cell/10-cm dish, and the medium of ΨCRIP/P131 producing retrovirus was changed from DMEM containing 10% bovine serum to α-MEM containing 12.5% inactivated horse serum, 12.5% inactivated fetal bovine serum, 2-Mercaptoethanol, and hydrocortisone for culture. On the day of transfection, the culture supernatant was filtered through a 0.20-µm filter, and polybrene was added at a final concentration of 8 µg/ml. Next, the recombinant retroviral vector produced in the supernatant was infected to stromal cells. After 4 hours, the culture supernatant was changed with fresh medium and further cultured for 2 days. Then, drug selection was carried out using 100 µg/ml hygromycin for 5 days for pBABE-hygro-hTERT; 1 µg/ml puromycin for 5 days for pBABE-puro-rasV12; and 1 mg/ml G418 for 5 days for pMFG-tsT-IRES-neo, respectively.

Infection was achieved using the 3 types of retroviral vectors in the following combinations: (1) control; (2) pBABE-hygro-hTERT vector alone; (3) pMFG-tsT-IRES-neo vector alone; (4) pBABE-puro-ras-V12 vector alone; (5) two vectors pMFG-tsT-IRES-neo and pBABE-hygro-hTERT; (6) two vectors pBABE-puro-ras-V12 and pBABE-hygro-hTERT; (7) two vectors pMFG-tsT-IRES-neo and pBABE-puro-ras-V12; and (8) three vectors pBABE-puro-ras-V12 and pMFG-tsT-IRES-neo and pBABE-hygro-hTERT.

The inventors have been keeping these viruses and cells, and will make such publicly available after obtaining patent for this application.

### Example 6: Differentiation potency of mesenchymal stem cell

Mesenchymal stem cells, which have the ability to differentiate into bone, cartilage, muscle, and the like as well as self-reproducing ability, were examined for their differentiation potency and ability to support blood stem cells.

Ilium puncture was conducted on a healthy adult human. Next, mononuclear cells were collected by specific gravity centrifugation and cultured in DMEM containing 10% inactivated fetal bovine serum overnight. Adherent cells were cultured from the next day. After 2 weeks, cells harvested upon T-E (trypsin-EDTA) were conserved as frozen primary mesenchymal stem cells. Then, the hTERT gene was introduced similarly to Example 1. The growth curves for primary mesenchymal stem cells and mesenchymal stem cells immortalized by introducing the hTERT gene were then compared with each other. The results are shown in Fig. 9. As shown in Fig. 9, the cell division of primary mesenchymal stem cells terminated (crisis) after 42 days (the number of generations, 17 (PD=17)). On the other hand, the immortalized mesenchymal stem cells kept the original cell division rate and could be passaged even after 270 days (the number of generations, 54 (PD=54)). Thus, a stable cell line was presumed to have been established.

Next, the prepared mesenchymal stem cell was examined for its pluripotency.
(1) First, differentiation into fat cells was induced using 1 µM dexamethasone, 60 µM indomethacin, 0.5 µM 3-isobutyl-1-methylxanthine and 5 µg/ml insulin. After culture for about one week, the cells were stained with Oil Red O (Fig. 10; lipid droplets are stained red).
(2) Then, cartilage differentiation was induced using 1 µM dexamethasone, 50 µg/ml ascorbate-2-phosphate, 6.25 µg/ml insulin, 6.25 µg/ml transferrin, 5.35 µg/ml selenic acid, 1.25 mg/ml linoleic acid and 10 ng/ml TGF-β. After a culture for 2-3 weeks, the cells were stained with Alcian blue. The chondroitin in the cartilage matrix (frozen section) was stained blue (Fig. 11).
(3) Furthermore, bone differentiation was induced using 1 µM dexamethasone, 50 µM ascorbate-2-phosphate and 10 mM β-glycerophosphate. After a culture for 2-3 weeks, the cells were subjected to von Kossa staining (deposition of minerals). The results are shown in Fig. 12.

### Example 7: Induction of differentiation of mesodermal stem cells into neural cells

### (1) Differentiation of mesodermal stem cells into neural stem cells:

Mesodermal stem cells (MSC) immortalized through the introduction of the hTERT gene as in the above Example were used as mesehchymal stem cells in the following experiments.

First, the immortalized cells were washed, and then the cells in the culture medium were collected upon enzymatic treatment (reagents: 0.05% trypsin/0.02% EDTA; at room temperature for 5 minutes). An equal volume of culture medium was added and pipetted for several times to disperse the cells into single cells. Then, the suspension was centrifuged at 900 g for 5 minutes. The precipitated cells were sucked up with a pipette. Then, the cells were further cultured at 37°C under 5% CO₂ in a suspension state in fresh culture medium 1 [50% DMEM (Dulbecco's modified essential medium)/50% F-12/1% FCS; 10 ng/ml basic fibroblast growth factor (bFGF) added every day; 10 ng/ml epidermal growth factor (EGF) added every day] or culture medium 2 [NPBM (Neural progenitor cell basal medium: Clonetics)/2% Neural survival factors (Clonetics)/10 ng/ml hEGF (human epidermal growth factor)/0.2% Gentamicin-amphotericin B/10 ng/ml hFGF (human fibroblast growth factor); 10 ng/ml basic fibroblast growth factor (bFGF) added every day; 10 ng/ml epidermal growth factor (EGF) added every day] in a culture container (non-treated polystyrene dish).

The differentiation of mesodermal stem cells into neural stem cells using this method was assessed via the disappearance of mesodermal cell markers SH2 and SH3 (the cells are originally negative with respect to the neural stem cell marker nestin) and that the appearance of the neural stem cell marker nestin (the cells turned positive with respect to this marker) (Fig. 13).

In addition, the morphology of the cultured mesodermal stem cells changed upon differentiation into neural stem cells. Specifically, the dispersed cells formed a neurosphere (Fig. 7). In addition, these neural stem cells were found to differentiate into neurons (NSE positive) and glial cells (GFAP positive) (Fig. 14).

### (2) Differentiation of mesodermal stem cells into neurons or glial cells [1]:

The culture medium of mesodermal stem cells cultured above was changed with a fresh culture medium [50% DMEM (Dulbecco's modified essential medium)/50% F-12/1% FSC] or culture medium 2 [NPBM (Neural progenitor cell basal medium: Clonetics)/2% Neural survival factors (Clonetics)/10 ng/ml hEGF (human epidermal growth factor)/0.2% Gentamicin-amphotericin B/10 ng/ml hFGF] and cultured for about 4 weeks. As a result, differentiation of mesodermal stem cells directly to neurons or glial cells (without conversion to neural stem cells) was successfully induced.

### (3) Differentiation of mesodermal stem cells into neurons or glial cells [2]:

Differentiation of mesodermal stem cells into neurons can also be induced by the following procedure. Induction of nerve was achieved according to the previous report (Journal of Neuroscience Research 61: 364-370, 2000). First, the cell density of MSC KY hTERT was adjusted. Specifically, the cells were pre-cultured on a 10-cm dish until 70-80% confluence, the supernatant was aspirated, washed with 5 ml of PBS, and 1 ml of trypsin-EDTA was added to collect the cells. The cells were pelleted by centrifugation at 210 g for 10 minutes, and then suspended at a cell density of 2x 10⁴ cells/ml in DMEM containing 10% fetal bovine serum. 2-ml aliquot of the solution was plated on each well (4x 10⁴ cells) of a 6-well plate. After 24 hours, DMEM containing 10% fetal bovine serum was removed, and the medium was changed with 2 ml of DMEM containing 1 mM of 2-ME and 10% fetal bovine serum (pre-induction). After another 24 hours, the serum was removed and the induction of nerve was carried out using the following three types of media: (1) 2 ml of DMEM containing 1 mM 2-ME; (2) 2 ml of DMEM containing 10 mM 2-ME; and (3) 2 ml of DMEM containing 200 µM butylated hydroxyanisole (BHA, Sigma) and 2% dimethylsulfoxide (DMSO, NACALAI). The morphology of the cells was observed after 24-hour induction using the media shown above.

As a result, using the medium of (1), the cytoplasm of MSC KY hTERT with a flat cytoplasm involuted and showed a morphology having bipolar or multipolar shapes contacting with adjacent cells. As compared with the results obtained using the medium of (1), more cells exhibiting almost the same morphology were observed using the medium of (2) or (3). These morphological changes were similar to those described in previous reports. The cells were further analyzed by immune-staining for neural makers. Solutions of 3 types of antibodies, i.e., antibodies against glial fibrillary acidic protein (GFAP; Cappel), myelin basic protein (MBP; Chemicon), and Neuron specific enolase (NSE; ARP) were diluted 50, 200, and 200 times, respectively, and used in the experiment. Further, the immuno-staining was carried out using Vectastain Elite ABC kit (VECTOR laboratories) and DAB substrate kit (VECTOR laboratories) as the substrate. The cells were fixed with 2% paraformaldehyde and endogenous peroxidase was inhibited with 0.3% hydrogen peroxide. The neural induction was carried out as described above and the immuno-staining was also performed 24 hours after the induction.

### Example 8: Evaluation of neural regeneration potency

The neural regeneration potency of neural cells obtained by the above-described method for inducing differentiation was assessed using a brain infarction model, a dementia model, a spinal cord injury model and a demyelination model. As a result, the cells were revealed to have regeneration potency comparable to that of neural stem cells isolated and cultured from the brain (Fig. 15).

### Example 9: Neural cell differentiation of neurosphere-like cells

Mesenchymal stem cells were immortalized by introducing the hTERT gene into the mesodermal stem cells according to the method described in the above Example (MSC-hTERT). Further, the hTERT gene was similarly introduced into stromal cells (Stroma-hTERT), PDF cells, Hela cells and HepG2 cells. Then, each of the cells were respectively suspended in Neural Progenitor basal medium and cultured in non-treated dishes. After 48 hours, the morphology of the cells was observed. The MSC-hTERT cells, Stroma-hTERT cells and PDF cells showed a sphere-like morphology (Fig. 16).

Furthermore, following the induction of differentiation of the above-mentioned respective cells into neural stem cells according to the method of the present invention (i.e., suspension culture for 1, 2, or 5 days in Neural Progenitor basal medium in non-treated dishes), total RNA was prepared from the cells and the expression of nestin was examined by RT-PCR.

Cells cultured merely in DMEM containing 10% FBS were used as controls. The induction of differentiation of mesodermal stem cells into stem cells by this method was determined as described in Example 8, by confirming the cells turning positive with respect to the neural stem cell marker nestin.

As a result, the expression of nestin was detected in the lanes containing MSC-hTERT, MSC, Stroma-hTERT and PDF cells cultured for 2 or 5 days (Fig. 17).

### Example 10: Differentiation into neurons and formation of neurosphere

The respective cells used in Example 10 were plated at a cell density of 5x 10⁴ cells/well in DMEM containing 10% FBS on a 6 well plate. After overnight culture, the medium was changed with DMEM containing 10% FBS and 1 mM β-ME, and the cells were further cultured overnight. Then, the medium was changed with DMEM containing 2% DMSO/200 µM butylated hydroxyanisole (BHA) for induction. After 4 days, the cells were collected and total RNA was extracted for RT-PCR.

Further, the above-mentioned respective cells were plated at a cell density of 5x 10⁵ cells/10-cm dish on non-coated dishes containing NPBM (FGF (20 ng/ml) and EGF (20 ng/ml)) medium to form a neurosphere. After 4 days, the cells were collected and plated at a cell density of 1x 10⁵ cells/well (6 well plates coated with poly-D-lysine/laminin) in NPBM (FGF, EGF(-)). After 10 days, the cells were collected and total RNA was prepared for RT-PCR.

In order to normalize the difference of cDNA concentration between the samples, the glyceraldehyde-3-phosphate dehydrogenase (GAPDH) gene was analyzed as an internal standard for normalization by the same method.

As a result, particularly strong expression of NF-M was observed for neurons differentiated from the MSC-hTERT cells (lane 6 in Fig. 18; BHA and DMSO) and neurons differentiated from the MSC-hTERT cells via neural stem cells (lane 13 in Fig. 18; NPBM(-) PDL/laminin).

### Example 11: Expression of nestin

A cell line was prepared by introducing into MSC-hTERT a vector that expresses EGFP when the host turns nestin positive. The differentiation into neural stem cells was induced *in vitro,* and then the cells were observed under a confocal laser microscope. As a result, nestin-positive MSC-hTERT cells were found to emit green fluorescence (Fig. 19).

Furthermore, the expression of nestin was also confirmed by RT-PCR (Fig. 20).

### Industrial Applicability

The present invention provides a method for inducing differentiation of mesodermal stem cells or ES cells into neural cells, a novel method which comprises obtaining a large number of cells by proliferating mesodermal stem cells and efficiently inducing differentiation of the cells into neural stem cells and neural cells; neural cells obtained by the methods; a composition for treating neurological diseases which comprises the neural cells; and a method for treating neurological diseases using the composition.

While the proliferation of cultured human normal mesodermal stem cells is limited in some degree under culture conditions, the mesodermal stem cells of the present invention into which the hTERT gene has been introduced can be extremely stably proliferated on a very large scale. Furthermore, unlike cells modified by the introduction of an oncogene or the like, the cells of the present invention exhibit no recognizable changes in cellular characteristics. Thus, immortalization of cells can be achieved without the loss of the original nature of the cells. In addition, the hTERT gene can be removed after sufficient proliferation of the cells.

The method of the present invention can contribute to the treatment of various neurological diseases, such as demyelination diseases in the central and peripheral nervous systems, degenerative diseases in the central and peripheral nervous systems, brain apoplexy (including brain infarction, brain hemorrhage, and subarachnoid hemorrhage), brain tumor, higher brain dysfunction including dementia, psychiatric diseases, epilepsy, traumatic neurological diseases (including head injury, brain contusion, and spinal cord injury), inflammatory diseases, infectious diseases (for example, Creutzfeldt-Jakob disease), and infarction of spinal cord. In addition, the present invention is considered to be applicable to neural transplantation and/or regeneration therapy for more general and diffuse cerebral and/or nerve damages. Specifically, the present invention can be applied to autotransplantation therapy for demyelination diseases in the central and peripheral nervous systems, degenerative diseases in the central and peripheral nervous systems, brain apoplexy (including brain infarction, brain hemorrhage, and subarachnoid hemorrhage), brain tumor, higher brain dysfunction including dementia, psychiatric diseases, epilepsy, traumatic neurological diseases (including head injury, brain contusion, and spinal cord injury), inflammatory diseases, infectious diseases (for example, Creutzfeldt-Jakob disease), and infarction of spinal cord.

Furthermore, the method for inducing differentiation of the present invention provides enables the understanding of the mechanism underlying the differentiation of mesodermal stem cells into neural cells. Once genes that direct such differentiation are identified and analyzed, a sufficient number of mesodermal stem cells can efficiently be transformed into neural cells using such genes. Thus, the present invention is greatly expected to enable "gene therapy" for promoting regeneration of neural tissues.

In addition, the method of the present invention is applicable to functional tests, toxicity tests, and cDNA cloning for drug discovery. Usually, a large number of neurons are required in an assay system for functional testing and toxicity testing in developing therapeutic drugs that act on the nerve or for searches of novel cDNAs that modify the function of human neural cells. Previously, it was extremely difficult to supply a sufficient number of human neurons. Further, hitherto immortalized cell lines could be relatively simply obtained from mouse cells or such. However, mostly, the neurons of human and cells derived from non-human animal species have a great difference (species specificity), and the experimental results obtained using animal cells or animal individuals have often been difficult to apply as alternatives of human cells to human. Using the cells and method of the present invention, a sufficient number of human neural cells can be easily prepared on a large scale to achieve all the experiments. Furthermore, such cells can be prepared to retain the nature and function of the original human neurons. Thus, according to the present invention, remarkable technological innovation can be achieved with respect to methods of functional testing, toxicity testing and cDNA cloning for drug discovery, methods that were previously unusable due to quantitative limitation.

For example, while functional tests and toxicity tests of compounds (drugs) have been performed by administering such compounds to experimental animals, such as rats, many of the test results did not correspond to human. Therefore, examinations using human samples have been desired in the art. Among all the human cells, human neural cells are hardly available. Thus, when a sufficient number of human neural cells can be obtained, functional tests and toxicity tests using human neural cells can be readily performed. Hence, such cells are very useful in drug discovery. A large number of human neural cells can be prepared by obtaining a large number of cells using the immortalized human MSC of the present invention and differentiating the prepared cells into neural cells according to the methods of the present invention.

The present invention provides a technique to differentiate mesodermal cells into neural cells. Further, by cloning genes whose expression level changes during the differentiation, genes regulating growth/differentiation can be identified. In addition, although a large number of cells will be required to achieve such study, the present invention has merits such as achievement of convenient gene cloning, due to the fact that a large number of immortalized human MSC can be prepared according to the method of the present invention.

## Claims

1. A method for inducing differentiation of a mesodermal stem cell into a neural cell, which comprises the steps of:
(a) providing an immortalized mesodermal stem cell by highly expressing or activating an immortalization gene; and
(b) inducing the differentiation of the immortalized mesodermal stem cell of step (a) directly into a neural cell without conversion to a neural stem cell by culturing the immortalized mesodermal stem cell in suspension, wherein the immortalized mesodermal stem cell is cultured in a cell culture medium composed of 50% DMEM, 50% F-12 and 1% FCS at 37°C, to which 10ng/ml bFGF and 10ng/ml EGF are added every day; thereafter the immortalized mesodermal stem cell is cultured in a cell culture medium composed of 50% DMEM, 50% F-12 and 1% FCS.

2. The method according to claim 1, wherein the immortalized mesodermal stem cell is cultured under 5% CO₂.

3. The method according to claim 1 or 2, wherein the immortalization gene is highly expressed or activated through the introduction of the immortalization gene into the mesodermal stem cell.

4. The method according to claim 3, wherein the mesodermal stem cell is immortalized by introducing the immortalization gene into the mesodermal stem cell using a retroviral vector.

5. The method according to claim 4, wherein the retroviral vector is pBabe.

6. The method according to any one of claims 1 to 5, wherein the immortalization gene of the neural cell can be or has been removed by a gene-removing treatment.

7. The method according to claim 6, wherein the gene-removing treatment of the immortalization gene is performed by sandwiching the immortalization gene within a pair of loxP sequences or loxP-like sequences and then treating with a recombinase.

8. The method according to any one of claims 1 to 7, wherein the immortalization gene is any one selected from the group of telomerase gene, genes derived from the telomerase, and genes which regulate the expression or activity of the telomerase.

9. The method according to any one of claims 1 to 8, wherein the mesodermal stem cell is derived from bone marrow fluid, umbilical blood, peripheral blood, skin, hair root cells of skin, and muscle tissue.

10. The method according to any one of claims 1 to 8, wherein the mesodermal stem cell is contained in a mononuclear cell fraction isolated from bone marrow fluid or umbilical blood collected from a vertebrate.

11. The method according to claim 10, wherein the mononuclear cell fraction can be prepared by subjecting the bone marrow fluid or umbilical blood collected from a vertebrate to density-gradient centrifugation in a solution at 2000 rpm for a sufficient time ensuring separation depending on the specific gravity, and then recovering a cell fraction within the range of specific gravity 1.07 g/ml to 1.1 g/ml.

12. The method according to any one of claims 1 to 11, wherein the culture is carried out at 33°C to 38°C.

13. The method according to any one of claims 1 to 12, which further comprises adding ischemic brain extract to a cell culture medium.

14. The method according to any one of claims 1 to 13, wherein the neural cell is selected from the group consisting of neuron, and glial cell.

15. The method according to any one of claims 1 to 14, wherein the mesodermal stem cell has the characteristics of SH2(+), SH3(+), SH4(+), CD29(+), CD44(+), CD14(-), CD34(-) and CD45(-).

## Patentansprüche

1. Verfahren zur Induktion der Differenzierung einer mesodermalen Stammzelle in eine Nervenzelle, wobei das Verfahren die folgenden Schritte umfasst:
(a) Bereitstellen einer immortalisierten mesodermalen Stammzelle durch starke Expression oder Aktivierung eines Immortalisationsgens; und
(b) Induzieren der Differenzierung der immortalisierten mesodermalen Stammzelle aus Schritt (a) direkt in eine Nervenzelle ohne Umwandlung in eine Nervenstammzelle durch Züchten der immortalisierten mesodermalen Stammzelle in Suspension,
wobei die immortalisierte mesodermale Stammzelle bei 37°C in einem Zellkulturmedium gezüchtet wird, das aus 50% DMEM, 50% F-12 und 1% FCS besteht, zu dem täglich 10 ng/ml bFGF und 10 ng/ml EGF zugeführt werden;
worauf die immortalisierte mesodermale Stammzelle in einem Zellkulturmedium gezüchtet wird, das aus 50% DMEM, 50% F-12 und 1 % FCS besteht.

2. Verfahren nach Anspruch 1, wobei die immortalisierte mesodermale Stammzelle unter 5% CO₂ gezüchtet wird.

3. Verfahren nach Anspruch 1 oder 2, wobei das Immortalisationsgen durch Einführen des Immortalisationsgens in die mesodermale Stammzelle stark exprimiert oder aktiviert wird.

4. Verfahren nach Anspruch 3, wobei die mesodermale Stammzelle durch Einführen des Immortalisationsgens in eine mesodermale Stammzelle unter Verwendung eines retroviralen Vektors immortalisiert wird.

5. Verfahren nach Anspruch 4, wobei der retrovirale Vektor pBabe ist.

6. Verfahren nach einem der Ansprüche 1 bis 5, wobei das Immortalisationsgen der Nervenzelle durch Genentfernungsbehandlung entfernt werden kann oder entfernt worden ist.

7. Verfahren nach Anspruch 6, wobei die Genentfernungsbehandlung des Immortalisationsgens durchgeführt wird, indem das Immortalisationsgen innerhalb eines Paares von IoxP-Sequenzen oder IoxP-ähnlichen Sequenzen plaziert ('*sandwiching*') und dann mit einer Rekombinase behandelt wird.

8. Verfahren nach einem der Ansprüche 1 bis 7, wobei das Immortalisationsgen ein beliebiges Gen ist, das ausgewählt ist aus der Gruppe aus Telomerasegen, von Telomerase abstammenden Genen und Genen, die die Expression oder Aktivität der Telomerase regulieren.

9. Verfahren nach einem der Ansprüche 1 bis 8, wobei die mesodermale Stammzelle aus Knochenmarkflüssigkeit, Nabelschnurblut, peripherem Blut, Haut, Haarwurzelzellen der Haut und Muskelgewebe stammt.

10. Verfahren nach einem der Ansprüche 1 bis 8, wobei die mesodermale Stammzelle in einer mononukleären Zellfraktion enthalten ist, die aus Knochenmarkflüssigkeit oder Nabelschnurblut isoliert ist, die/das einem Wirbeltier entnommen wurde.

11. Verfahren nach Anspruch 10, wobei die mononukleäre Zellfraktion zubereitet werden kann, indem die Knochenmarkflüssigkeit oder das Nabelschnurblut, die/das von einem Wirbeltier entnommen wurde, in einer Lösung bei 2000 UpM einer Dichtegradientenzentrifugation für einen Zeitraum unterzogen wird, der ausreicht, um die Trennung in Abhängigkeit von dem spezifischen Gewicht zu gewährleisten, und dann eine Zellfraktion innerhalb des Bereichs des spezifischen Gewichts von 1,07 g/ml bis 1,1 g/ml gewonnen wird.

12. Verfahren nach einem der Ansprüche 1 bis 11, wobei die Kultur bei 33°C bis 38°C ausgeführt wird.

13. Verfahren nach einem der Ansprüche 1 bis 12, das des Weiteren das Hinzufügen von ischämischem Gehirnextrakt zu dem Zellkulturmedium umfasst.

14. Verfahren nach einem der Ansprüche 1 bis 13, wobei die Nervenzelle ausgewählt ist aus der Gruppe bestehend aus Neuronen- und Gliazellen.

15. Verfahren nach einem der Ansprüche 1 bis 14, wobei die mesodermale Stammzelle die Eigenschaften von SH2(+), SH3(+), SH4(+), CD29(+), CD44(+), CD14(-), CD34(-) und CD45(-) hat.

## Revendications

1. Méthode pour induire la différenciation d'une cellule souche mésodermique en une cellule neuronale, qui comprend les étapes consistant à :
(a) fournir une cellule souche mésodermique immortalisée par surexpression ou activation d'un gène d'immortalisation ; et
(b) induire la différenciation de la cellule souche mésodermique immortalisée de l'étape (a) directement en une cellule neuronale sans conversion en une cellule souche neuronale en cultivant la cellule souche mésodermique immortalisée en suspension,
dans laquelle la cellule souche mésodermique immortalisée est cultivée dans un milieu de culture cellulaire composé de 50% DMEM, 50% F-12 et 1% FCS à 37°C, dans lequel sont ajoutés quotidiennement 10 ng/ml de bFGF et 10 ng/ml d'EGF; par la suite la cellule souche mésodermique immortalisée est cultivée dans un milieu de culture cellulaire composé de 50% DMEM, 50% F-12 et 1% FCS.

2. Méthode selon la revendication 1, dans laquelle la cellule souche mésodermique immortalisée est cultivée sous 5% CO₂.

3. Méthode selon la revendication 1 ou 2, dans laquelle le gène d'immortalisation est surexprimé ou activé par l'introduction du gène d'immortalisation dans la cellule souche mésodermique.

4. Méthode selon la revendication 3, dans laquelle la cellule souche mésodermique est immortalisée par l'introduction du gène d'immortalisation dans la cellule souche mésodermique en utilisant un vecteur rétroviral.

5. Méthode selon la revendication 4, dans laquelle le vecteur rétroviral est pBabe.

6. Méthode selon l'une quelconque des revendications 1 à 5, dans laquelle le gène d'immortalisation de la cellule neuronale peut être ou a été éliminé par un traitement d'élimination de gène.

7. Méthode selon la revendication 6, dans laquelle le traitement d'élimination de gène du gène d'immortalisation est effectué en prenant en sandwich le gène d'immortalisation dans une paire de séquences loxP ou de séquences de type loxP puis en traitant avec une recombinase.

8. Méthode selon l'une quelconque des revendications 1 à 7, dans laquelle le gène d'immortalisation est n'importe quel gène choisi parmi le groupe du gène de la télomérase, des gènes dérivés de la télomérase, et des gènes qui régulent l'expression ou l'activité de la télomérase.

9. Méthode selon l'une quelconque des revendications 1 à 8, dans laquelle la cellule souche mésodermique est obtenue à partir du fluide de la moelle osseuse, du sang ombilical, du sang périphérique, de la peau, des cellules de racines de cheveux de la peau, et du tissu musculaire.

10. Méthode selon l'une quelconque des revendications 1 à 8, dans laquelle la cellule souche mésodermique est contenue dans une fraction de cellules mononucléaires isolée à partir du fluide de la moelle osseuse ou du sang ombilical prélevé sur un vertébré.

11. Méthode selon la revendication 10, dans laquelle la fraction de cellules mononucléaires peut être préparée en soumettant le fluide de moelle osseuse ou le sang ombilical prélevé sur un vertébré à une centrifugation en gradient de densité dans une solution à 2000 tours par minute pendant une durée suffisante pour assurer la séparation en fonction du poids spécifique, et en récupérant ensuite une fraction de cellules dans la gamme du poids spécifique de 1,07 g/ml à 1,1 g/ml.

12. Méthode selon l'une quelconque des revendications 1 à 11, dans laquelle la culture est effectuée à une température de 33°C jusqu'à 38°C.

13. Méthode selon l'une quelconque des revendications 1 à 12, qui comprend en outre l'ajout d'extrait de cerveau ischémique dans un milieu de culture cellulaire.

14. Méthode selon l'une quelconque des revendications 1 à 13, dans laquelle la cellule neuronale est choisie parmi le groupe constitué du neurone, et de la cellule gliale.

15. Méthode selon l'une quelconque des revendications 1 à 14, dans laquelle la cellule souche mésodermique présente les caractéristiques de SH2(+), SH3(+), SH4(+), CD29(+), CD44(+), CD14(-), CD34(-) et CD45(-).
